# EUROPEAN PATENT APPLICATION

(11) **EP 2 674 501 A1**
(43) Date of publication of application: **18.12.2013**
(21) Application number: 12171941.3
(22) Date of filing: 14.06.2012
(51) Int. Cl.: C12Q 1/68

(54) **Method for detecting and identifying enterohemorrhagic Escherichia coli**

(71) Applicant: Agence nationale de sécurité sanitaire de l'alimentation,de l'environnement et du travail, 94701 Maisons-Alfort Cedex (FR)
(72) Inventor: Fach, Patrick, 94200 Charenton Le Pont (FR); Delannoy, Sabine, 94230 Cachan (FR); Beutin, Lothar, 14163 Berlin (DE)
(74) Representative: Vialle-Presles, Marie José

(57) **Abstract**

The invention relates to methods for predicting whether a sample contains enterohemorrhagic *Escherichia coli* (EHEC) of at least one of EHEC O157:[H7], O145:[H28], O103:[H2], O111:[H8], O121:[H19], 026:[H11] or 045:[H2] serotypes, and for identifying said serotypes, through detection of markers genes including *Z2098*, *espK*, *espN*, *espM1* and *espV* and/or through detection of serotype-specific CRISPR sequences.

SEQUENCE LISTING

<110>  AGENCE NATIONALE DE SECURITE SANITAIRE DE L'ALIMENTATION,
       DE L'ENVIRONNEMENT ET DU TRAVAIL

<120>  Method for detecting and identifying enterohemorrhagic
       Escherichia coli

<130>  MJP-11-F1159-8

<160>  94

<170>  PatentIn version 3.5

<210>  1
<211>  334
<212>  DNA
<213>  Escherichia coli

<400>  1
gggtgttcta ccgcaggggc ggggaattct aagtgatatc catcatcgca tccagtgcgc      60

ccggtttatc cccgctgatg cggggaacac agcggcacgc tggattgaac aaatccctgg     120

gccggtttat ccccgctggc gcggggaaca ctttatacac ggatcctgtg tgccgtggac     180

cgccggttta tccccgctgg cgcggggaac acgagctggc gatagccctg aatgaactgg     240

cagacggtta atccccgctg gcgcggggaa cacaaaccga aacacacgat caatccgaat     300

atgagcggtt tatccccgct ggcgcgggga acac                                 334


<210>  2
<211>  212
<212>  DNA
<213>  Escherichia coli

<400>  2
gggtgttcta ccgcaggggc ggggaattct aagtgatatc catcatcgca tccagtgcgc      60

ccggtttatc cccgctgatg cggggaacac agcggcacgc tggattgaac aaatccctgg     120

gccggtttat ccccgctggc gcggggaaca caaaccgaaa cacacgatca atccgaatat     180

gagcggttta tccccgctgg cgcggggaac ac                                   212


<210>  3
<211>  212
<212>  DNA
<213>  Escherichia coli

<400>  3
gggtgttcta ccgcaggggc ggggaattct aagtgatatc catcatcgca tccagtgcgc      60

ccggtttatc cccgctgatg cggggaacac agcggcacgc tggattgaac aaatccctgg     120

gccggtttat ccccgctggc gcggggaaca ctttggtgac agtttttgtc actgttttgg     180

tgacggttta tccccgctgg cgcggggaac ac                                   212


<210>  4
<211>  273

<212>  DNA
<213>  Escherichia coli

<400>  4
gggtgttcta ccgcaggggc ggggaattct aagtgatatc catcatcgca tccagtgcgc      60

ccggtttatc cccgctgatg cggggaacac agcggcacgc tggattgaac aaatccctgg     120

gccggtttat ccccgctggc gcggggaaca caaaccgaaa cacacgatca atccgaatat     180

gagcggttta tccccgctgg cgcggggaac actttggtga cagtttttgt cactgttttg     240

gtgacggttt atccccgctg gcgcggggaa cac                                  273


<210>  5
<211>  272
<212>  DNA
<213>  Escherichia coli

<400>  5
tggtttatcc ccactggcgc ggggaacttg agccctgcca gaatggggcc tcttttgtac      60

ccggtttatc cccgctgccg cggggaacac atttactgat tgtaccaggt aggaaaagat     120

cgcggtttat ccccgctggc gcggggaact catgattgtc gattttttcc gacaggattt      180

tgcggtttat ccccgctggc gcggggaact cgtcacactg ccgtgcatta ttacagccag     240

gcgcggttta tccccgctgg cgcggggaac tc                                   272


<210>  6
<211>  395
<212>  DNA
<213>  Escherichia coli

<400>  6
tggtttatcc ccgctggcgc ggggaactcg acagaacggc ctcagtagtc tcgtcaggct      60

ccggtttatc cccgctggcg cggggagctc tacgtgaaga atatttgcaa cacccgcaag     120

aacggtttat ccccgctggc gcggggaaca ctccaacctt ccatgagata cgcgcattag     180

cggcggttta tccccgctgg cgcggggaac accgtgaccg cctgtacacg ctgtaatggc     240

tcaccggttt atccccgctg gcgcggggaa cacacactat ccgggcggta ttacgcccaa     300

atatccggtt tatccccgct ggcgcgggga acacctgccg ggtgaaacca ctcgcggcag     360

atcttgcggt ttatccccgc tggcgcgggg aacac                                395


<210>  7
<211>  334
<212>  DNA
<213>  Escherichia coli

<400>  7
gggagttcta ccgcaggggc ggggaattct aagtgatatc catcatcgca tccagtgcgc      60

ccggtttatc cccgctggcg cggggaacac tcacgcgctg ttacccagtc cctttttttc     120

agcggtttat ccccgctggc gcggggaaca ctacaggaaa gaatccgcca acggcgacag     180

gggcggttta tccccgctgg cgcggggaac acgaatatcc acccgtcctg tattccgcca 240

attgcggttt atccccgctg gcgcggggaa cactaccaaa ccggaatctt tccatataac 300

ggcggcggtt tatccccgct ggcgcgggga acac 334

<210> 8
<211> 273
<212> DNA
<213> Escherichia coli

<400> 8
gggagttcta ccgcaggggc ggggaattct aagtgatatc catcatcgca tccagtgcgc 60

ccggtttatc cccgctgacg cggggaacac aaaaccaaac ttctccataa attccatagc 120

cgcggtttat ccccgctggc gcggggaaca cgagtctatc agcgacacta ccggcaatag 180

cgacggttta tccccgctgg cgcggggaac acctatagcg ccacgttccg agcgctgcga 240

gctgcggttt atccccgctg gcgcggggaa cac 273

<210> 9
<211> 822
<212> DNA
<213> Escherichia coli

<400> 9
tggtttatcc ccgctggcac ggggaactcg cctgacattg cagacgttac aaattgagag 60

gcggtttatc cccgctggcg cggggaactc taatcacgtt ttagcgcgcc ctcgtccggt 120

ttcggtttat ccccgctggc gcggggaact catcttcttg ttcaggaacg tcagtagagg 180

tgtcggttta tccccgctgg cgcggggaac tcagcattaa cccccaccag ctcgacgtgt 240

gtggcggttt atccccgctg gcgcggggaa ctcgcgggcg ttaacgcggt gatactgttt 300

gacggcggtt tatccccgct ggcgcgggga actcaccgag cgccgctggg aggcgtatct 360

cacgttcggt ttatccccgc tggcgcgggg aactctgaat gatcggaaag acgctgcaaa 420

ggcaatgcgg tttatccccg ctggcgcggg gaactcatta aaggattatt ttgatgagtc 480

tgaaaaatcg gtttatcccc gctggcgcgg ggaactctga ttttggtggc gggggtgact 540

gggggaatac ggtttatccc cgctggcgcg gggaactcta ccggtgtgaa ttacagcacc 600

accgccaccc cggtttatcc ccgctggcgc ggggaactcc aaacaggtcg acatgtttgg 660

ctaacagcta acggtttatc cccgctggcg cggggaactc cgataacaca gggccgtcaa 720

tgcggccctt ttcggtttat ccccgctggc gcggggaact cataccgggt tttgctccag 780

cagcgccagt tcctggttta tccccgctgg cgcggggaac tc 822

<210> 10
<211> 578
<212> DNA
<213> Escherichia coli

<400> 10

tggtttatcc ccgctggcgc ggggagctca acatcggaaa cggcttcgcg gcggcggcgt 60

ccggtttatc cccgctggcg cggggaactc gacagaacgg cctcagtagt ctcgtcaggc 120

tccggtttat ccccgctggc gcggggagct ctacgtgaag aatatttgca acacccgcaa 180

gaacggttta tccccgctgg cgcggggagc tcggcatagc caggctgatc cggcgacggc 240

cttacggttt atccccgctg gcgcggggaa cacaatcgtg tgtaaattcg cggcggctcc 300

actggcggtt tatccccgct ggcgcgggga acactggtcg aaatatagac agcatgttcc 360

gtaccacggt ttatccccgc tggcgcgggg aacacacact atccgggcgg tattacgccc 420

aaatatccgg tttatccccg ctggcgcggg gaacacctgc cgggtgaaac cactcgcggc 480

agatcttgcg gtttatcccc gctggcgcgg ggaacacact actgtcggta gctgggagga 540

tgaggagatc ggtttatccc cgctggcgcg gggaacac 578

<210> 11
<211> 21
<212> DNA
<213> Escherichia coli

<400> 11
gggaacacaa accgaaacac a 21

<210> 12
<211> 19
<212> DNA
<213> Escherichia coli

<400> 12
cttagtgtgt tccccgcgc 19

<210> 13
<211> 24
<212> DNA
<213> Escherichia coli

<400> 13
gaacactttg gtgacagttt ttgt 24

<210> 14
<211> 19
<212> DNA
<213> Escherichia coli

<400> 14
cttagtgtgt tccccgcgc 19

<210> 15
<211> 21
<212> DNA
<213> Escherichia coli

<400> 15
gaacacaaac cgaaacacac g 21

<210>  16
<211>  22
<212>  DNA
<213>  Escherichia coli

<400>  16
ataaaccgtc accaaaacag tg                                                22


<210>  17
<211>  20
<212>  DNA
<213>  Escherichia coli

<400>  17
gaacttgagc cctgccagaa                                                   20


<210>  18
<211>  21
<212>  DNA
<213>  Escherichia coli

<400>  18
accgcgatct tttcctacct g                                                 21


<210>  19
<211>  18
<212>  DNA
<213>  Escherichia coli

<400>  19
gtgaccgcct gtacacgc                                                     18


<210>  20
<211>  21
<212>  DNA
<213>  Escherichia coli

<400>  20
cggatatttg ggcgtaatac c                                                 21


<210>  21
<211>  19
<212>  DNA
<213>  Escherichia coli

<400>  21
ctgccgcgag tggtttcac                                                    19


<210>  22
<211>  22
<212>  DNA
<213>  Escherichia coli

<400>  22
cggggaacac tacaggaaag aa                                                22


<210>  23

<211>  21
<212>  DNA
<213>  Escherichia coli

<400>  23
ggcggaatac aggacgggtg g                                                21


<210>  24
<211>  21
<212>  DNA
<213>  Escherichia coli

<400>  24
gagtctatca gcgacactac c                                                21


<210>  25
<211>  18
<212>  DNA
<213>  Escherichia coli

<400>  25
aaccgcagct cgcagcgc                                                    18


<210>  26
<211>  18
<212>  DNA
<213>  Escherichia coli

<400>  26
ggaactcacc gagcgccg                                                    18


<210>  27
<211>  23
<212>  DNA
<213>  Escherichia coli

<400>  27
gcctttgcag cgtctttccg atc                                              23


<210>  28
<211>  22
<212>  DNA
<213>  Escherichia coli

<400>  28
acaatcgtgt gtaaattcgc gg                                               22


<210>  29
<211>  21
<212>  DNA
<213>  Escherichia coli

<400>  29
gataaaccgt ggtacggaac a                                                21


<210>  30
<211>  20
<212>  DNA

<213>  Escherichia coli

<400>  30
tgaaaccact cgcggcagat                                                    20


<210>  31
<211>  22
<212>  DNA
<213>  Escherichia coli

<400>  31
ataaaccgat ctcctcatcc tc                                                 22


<210>  32
<211>  23
<212>  DNA
<213>  Escherichia coli

<400>  32
cgatcaatcc gaatatgagc ggt                                                23


<210>  33
<211>  24
<212>  DNA
<213>  Escherichia coli

<400>  33
cactgttttg gtgacggttt atcc                                               24


<210>  34
<211>  24
<212>  DNA
<213>  Escherichia coli

<400>  34
acaaaaactg tcaccaaagt gttc                                               24


<210>  35
<211>  21
<212>  DNA
<213>  Escherichia coli

<400>  35
tggggcctct tttgtacccg g                                                  21


<210>  36
<211>  25
<212>  DNA
<213>  Escherichia coli

<400>  36
tgtaatggct caccggttta tcccc                                              25


<210>  37
<211>  20
<212>  DNA
<213>  Escherichia coli

<400>  37
tccgccaacg gcgacagggg                                                    20


<210>  38
<211>  22
<212>  DNA
<213>  Escherichia coli

<400>  38
tcggaacgtg gcgctatagg tg                                                 22


<210>  39
<211>  25
<212>  DNA
<213>  Escherichia coli

<400>  39
ctgggaggcg tatctcacgt tcggt                                              25


<210>  40
<211>  27
<212>  DNA
<213>  Escherichia coli

<400>  40
tgctgtctat atttcgacca gtgttcc                                            27


<210>  41
<211>  25
<212>  DNA
<213>  Escherichia coli

<400>  41
ccagctaccg acagtagtgt gttcc                                              25


<210>  42
<211>  21
<212>  DNA
<213>  Escherichia coli

<400>  42
ctgaaaagag ccagaacgtg c                                                  21


<210>  43
<211>  22
<212>  DNA
<213>  Escherichia coli

<400>  43
tgcctaagat cattacccgg ac                                                 22


<210>  44
<211>  26
<212>  DNA
<213>  Escherichia coli

<400>  44
gcagrcatca aaagcgaaat cacacc                                             26

<210>  45
<211>  26
<212>  DNA
<213>  Escherichia coli

<400>  45
tcgtttggta actgtggcag atactc                                          26


<210>  46
<211>  29
<212>  DNA
<213>  Escherichia coli

<400>  46
gacatatttg tttatgtcat caggagcgg                                       29


<210>  47
<211>  26
<212>  DNA
<213>  Escherichia coli

<400>  47
cctcaggata tggatggcct actggc                                          26


<210>  48
<211>  25
<212>  DNA
<213>  Escherichia coli

<400>  48
gcgctctatc cgctttaatg ttaac                                           25


<210>  49
<211>  28
<212>  DNA
<213>  Escherichia coli

<400>  49
ccatccatga atatctttag tactctgc                                        28


<210>  50
<211>  23
<212>  DNA
<213>  Escherichia coli

<400>  50
tcaggttcct cgtctgatgc cgc                                             23


<210>  51
<211>  23
<212>  DNA
<213>  Escherichia coli

<400>  51
ctggttcagg cctggagcag tcc                                             23

<210>  52
<211>  29
<212>  DNA
<213>  Escherichia coli

<400>  52
tttgtyactg tsacagcwga agcyttacg                                          29


<210>  53
<211>  26
<212>  DNA
<213>  Escherichia coli

<400>  53
ccccagttca rwgtragrtc macrtc                                             26


<210>  54
<211>  22
<212>  DNA
<213>  Escherichia coli

<400>  54
taactgctat acctccgcgc cg                                                 22


<210>  55
<211>  26
<212>  DNA
<213>  Escherichia coli

<400>  55
attcagatag aagaagcgcg ggccag                                             26


<210>  56
<211>  29
<212>  DNA
<213>  Escherichia coli

<400>  56
aatgctctcg gcaatcgaat ccttgactc                                          29


<210>  57
<211>  28
<212>  DNA
<213>  Escherichia coli

<400>  57
tgcttaccgt ctccagtata cagccgct                                           28


<210>  58
<211>  28
<212>  DNA
<213>  Escherichia coli

<400>  58
cttgcaacac gttacgctgc cgagtatt                                           28


<210>  59
<211>  31

<212>  DNA
<213>  Escherichia coli

<400>  59
ctggatgatc tcagtgggcg ttcttatgta a                                    31


<210>  60
<211>  27
<212>  DNA
<213>  Escherichia coli

<400>  60
tcgtcaggca ctgtctgaaa ctgctcc                                         27


<210>  61
<211>  24
<212>  DNA
<213>  Escherichia coli

<400>  61
ggtgaaggag ytggcgaagg cgtc                                            24


<210>  62
<211>  23
<212>  DNA
<213>  Escherichia coli

<400>  62
ccggtggatt tggatgggtt acg                                             23


<210>  63
<211>  22
<212>  DNA
<213>  Escherichia coli

<400>  63
tgtgaacctc tctggcatgg ag                                              22


<210>  64
<211>  24
<212>  DNA
<213>  Escherichia coli

<400>  64
taaagttggt agattgtgac tggc                                            24


<210>  65
<211>  20
<212>  DNA
<213>  Escherichia coli

<400>  65
gccgttacca tctatgatgc                                                 20


<210>  66
<211>  20
<212>  DNA
<213>  Escherichia coli

<400>  66
tgtaaacgcg catgaagctg                                                   20


<210>  67
<211>  28
<212>  DNA
<213>  Escherichia coli

<400>  67
agcgcgtaaa caaaacttcg gcatggtg                                          28


<210>  68
<211>  19
<212>  DNA
<213>  Escherichia coli

<400>  68
agtggtacgg gtaaaaccg                                                    19


<210>  69
<211>  20
<212>  DNA
<213>  Escherichia coli

<400>  69
agtatcagcg tgtaactggc                                                   20


<210>  70
<211>  25
<212>  DNA
<213>  Escherichia coli

<400>  70
tggaaatcag catccgagga atgcc                                             25


<210>  71
<211>  24
<212>  DNA
<213>  Escherichia coli

<400>  71
tttatcctgt cacacagcag agtg                                              24


<210>  72
<211>  26
<212>  DNA
<213>  Escherichia coli

<400>  72
acaaaaccca gttataagcc aaatcg                                            26


<210>  73
<211>  28
<212>  DNA
<213>  Escherichia coli

<400>  73

tcatcatccc agtcaggctt cccagagc 28

<210> 74
<211> 24
<212> DNA
<213> Escherichia coli

<400> 74
catgttgttg atgtaagtat gcag 24

<210> 75
<211> 23
<212> DNA
<213> Escherichia coli

<400> 75
aagttcacaa gtacattacc cgg 23

<210> 76
<211> 26
<212> DNA
<213> Escherichia coli

<400> 76
acgctggctt gtcttttatg ggaccg 26

<210> 77
<211> 22
<212> DNA
<213> Escherichia coli

<400> 77
gacagtcttc gattgccttt gc 22

<210> 78
<211> 28
<212> DNA
<213> Escherichia coli

<400> 78
gcagatttgc atcattttct aaattcac 28

<210> 79
<211> 28
<212> DNA
<213> Escherichia coli

<400> 79
agagccttgt tgatgccctg tttcgttc 28

<210> 80
<211> 28
<212> DNA
<213> Escherichia coli

<400> 80
aagttctatt aatcatgtra taatgggg 28

<210> 81
<211> 26
<212> DNA
<213> Escherichia coli

<400> 81
acttgtcygc aagcaagttt gctatg 26


<210> 82
<211> 28
<212> DNA
<213> Escherichia coli

<400> 82
gctctttgtg atrgtatgag ccgggatg 28


<210> 83
<211> 25
<212> DNA
<213> Escherichia coli

<400> 83
cttaggagag cgagacgtaa gaatg 25


<210> 84
<211> 26
<212> DNA
<213> Escherichia coli

<400> 84
tgaataaaga tactcaccta actctg 26


<210> 85
<211> 31
<212> DNA
<213> Escherichia coli

<400> 85
ttccttcctg ttatcttatc tgtatcatca g 31


<210> 86
<211> 22
<212> DNA
<213> Escherichia coli

<400> 86
gcaataattg actctgattg cc 22


<210> 87
<211> 21
<212> DNA
<213> Escherichia coli

<400> 87
gctgctgcgg taaaatttac t 21


<210> 88

<211>  24
<212>  DNA
<213>  Escherichia coli

<400>  88
tacgctgatc accatgcctg gtgc                                              24


<210>  89
<211>  20
<212>  DNA
<213>  Escherichia coli

<400>  89
tagcgggaca attgtcacgg                                                   20


<210>  90
<211>  23
<212>  DNA
<213>  Escherichia coli

<400>  90
gtctttcgga gaaacattct gcc                                               23


<210>  91
<211>  24
<212>  DNA
<213>  Escherichia coli

<400>  91
atattgatga cagcgtatgg gccg                                              24


<210>  92
<211>  24
<212>  DNA
<213>  Escherichia coli

<400>  92
gatggcagat aataacgaag caac                                              24


<210>  93
<211>  20
<212>  DNA
<213>  Escherichia coli

<400>  93
cagccgttga agcatcagcg                                                   20


<210>  94
<211>  26
<212>  DNA
<213>  Escherichia coli

<400>  94
agcagcattc ttgcagaccc ttacgg                                            26

## Description

The invention relates to the identification of Shiga toxin producing *E. coli* (STEC) that constitute a severe risk for human health.

Shiga toxin-producing *Escherichia coli* (STEC) are a diverse group of E. *coli* belonging to over 400 *E. coli* O:H serotypes, some of which cause outbreaks and sporadic cases of foodborne illness ranging from diarrhoea to hemorrhagic colitis (HC) and the haemolytic uremic syndrome (HUS). According to their human pathogenicity the latter strains were also designated as enterohaemorrhagic *E. coli* (EHEC) (Levine 1987, Nataro and Kaper 1998). Numerous cases of HC and HUS have been attributed to EHEC serotype O157:H7 strains, but it has now been recognized that other serotypes of STEC belong to the EHEC group.

Hence, cumulative evidence from numerous countries indicates that up to 30-60% of human STEC infections are caused by non-O157 STEC and that as few as five to seven "priority" serotypes of STEC are implicated in outbreaks and sporadic cases of HC and HUS. These comprise serotypes O26:[H11], O45:[H2], 0103:[H2], O111:[H8], O121:[H19], O145:[H28], O157:[H7] and their non-motile derivatives. In addition, an unusual strain of O104:[H4] has been associated with the largest outbreak of HC and HUS worldwide in 2011 (Scheutz et al., 2011; Frank et al., 2011; Struelens et al., 2011; Gault et al., 2011).

Consequently, many jurisdictions are considering implementation of food inspection programs to safeguard the public from these STEC strains with high virulence for humans. A rational approach for detection of these enterohaemorrhagic *E. coli* (EHEC) strains, as part of a risk-based food inspection program, requires clear definition of the hallmark characteristic of priority STEC (e.g. serogroup, serotypes, virulence and other markers) and effective approaches to detect these pathogenic STEC in foods. Detection of non-O157 EHEC is particularly challenging because, they have no specific characteristics that distinguish them from the large number of harmless commensal *E. coli* that share the same niches. A seropathotype classification has been proposed by Karmali et al. (2003) as a framework to identify the most important O-serogroups involved in food-borne outbreaks, based on severity of disease, frequency and association with outbreaks, but the reasons for the difference in virulence between the various STEC strains remains unclear. It is probable that this difference is due to differences in the pattern of virulence genes possessed by STEC strains and studies are needed to substantiate this and to identify appropriate molecular markers.

Techniques exist to determine the presence of a STEC contamination in a sample by for instance detecting the presence of the *stx1*/*stx2* genes and the *eae* gene located on the LEE (locus of enterocyte effacement), a locus that was first identified in enteropathogenic *E. coli* (EPEC). But the genetic basis of STEC pathogenicity is a lot more complex than the presence or absence of one or both of these genes. In a complex sample (e.g. food, fecal, environmental samples), which may comprise a mixture of strains (e.g. a mix of STEC and EPEC strains), the presence of the *stx1*/*2* and *eae* genes is not indicative of the presence of an EHEC in this sample.

However, given that some STEC strains can cause very serious health problems in humans, the detection of a STEC strain in a food product leads to discarding said product, even though it is likely this STEC does not pose a threat to human health. This results in a large amount of wastage due to lack of discrimination between non-pathogenic STEC strains and EHEC strains.

It has been proposed to use, in addition to the *stx1*/*stx2* and *eae* markers, other genetic markers in order to selectively detect EHEC strains and differentiate them from non-pathogenic STEC strains. For instance, PCT WO 2011/018762 describes a method involving the combined detection of the genes *stx1, stx2, eae, nleB* and *espK* to predict the presence of EHEC in a sample.

However, there is still a need of reliable tests allowing a discriminative screening for the presence of EHEC, including non-O157 EHEC, and a specific detection of the EHEC serotypes involved, in particular in case of the "top seven" serotypes O26:[H11], O45:[H2], O103:[H2], O111:[H8], O121:[H19], O145:[H28], O157:[H7].

The inventors have now identified discriminative genetic markers associated with several STEC strains constituting a severe risk for human health. In particular, they have identified genetic markers located within CRISPRs (Clustered Regularly Interspaced Short Palindromic Repeats) sequences of EHEC strains with high virulence for humans.

CRISPRs are present within the genomes of many bacterial species, including E. *coli.* They consist of tandem sequences containing direct repeats of 21 to 47 bp long and separated by spacers of similar size. Spacers are derived from foreign nucleic acids, such as phages or plasmids, and it has been hypothesized that they can protect bacteria from subsequent infection by homologous phages and plasmids.

The inventors have sequenced the CRISPR loci of various EHEC strains which are associated with the world's most frequent clinical cases, and have identified different spacers that can be used for a specific identification of the EHEC serotypes O157:[H7], O145:[H28], O103:[H2], O111:[H8], O121:[H19], O45:[H2], O26:[H11], O104:[H4] and their non motile derivatives, which are responsible for the majority of EHEC infections in humans.

Therefore, an object of the present invention is a method for identifying the serotype(s) of EHEC suspected to be present in a sample, wherein said method comprises detecting the presence or the absence, in said sample or DNA isolated therefrom, of the following *E. coli* CRISPRs sequences:
a) CRISPRs sequences for identifying EHEC O157:[H7] wherein said CRISPRs sequences are selected among:
   - the CRISPRs sequences SEQ ID NO: 1, SEQ ID NO: 2, and SEQ ID NO: 3, wherein the presence of one or more of said sequences SEQ ID NO: 1-3 is indicative of the presence of EHEC O157:[H7]; and/or
   - the CRISPR sequence SEQ ID NO: 4, wherein the presence of said CRISPR sequence is indicative of the presence of EHEC O157:[H7]; and
b) a CRISPR sequence for identifying EHEC O145:[H28], wherein said CRISPR sequence is the sequence SEQ ID NO: 5, and wherein the presence of said CRISPR sequence is indicative of the presence of EHEC O145:[H28]; and
c) a CRISPR sequence for identifying EHEC O111:[H8], wherein said CRISPR sequence is the sequence SEQ ID NO: 6, and wherein the presence of said CRISPR sequence is indicative of the presence of EHEC O111:[H8]; and
d) a CRISPR sequence for identifying EHEC O121:[H19], wherein said CRISPR sequence is the sequence SEQ ID NO: 7, and wherein the presence of said CRISPR sequence is indicative of the presence of EHEC O121:[H19]; and
e) a CRISPR sequence for identifying EHEC O103:[H2] and/or EHEC O45:[H2], wherein said CRISPR sequence is the sequence SEQ ID NO: 8, and wherein the presence of said CRISPR sequence is indicative of the presence of EHEC O103:[H2] and/or of EHEC O45:[H2]; and
f) a CRISPR sequence for identifying EHEC O104:[H4], wherein said CRISPR sequence is the sequence SEQ ID NO: 9, and wherein the presence of said CRISPR sequence is indicative of the presence of EHEC O104:[H4]; and
g) a CRISPR sequence for identifying EHEC O26:[H11], wherein said CRISPR sequence is the sequence SEQ ID NO: 10, and wherein the presence of said CRISPR sequence is indicative of the presence of EHEC O26:[H11].

According to a preferred embodiment of the invention, said method comprises performing a PCR assay on said sample or DNA isolated therefrom, with primers designed for amplifying said CRISPR sequences, and checking for the presence of the corresponding amplification products.

Preferably, said PCR assay is performed with a combination of primers comprising:
a) primers for detecting EHEC O157:[H7], wherein said primers consist of:
   - a set of primers targeting both the CRISPR sequences SEQ ID NO: 1 and SEQ ID NO: 2, wherein said primers are defined by the following sequences:
      GGGAACACAAACCGAAACACA (SEQ ID NO: 11)
      CTTAGTGTGTTCCCCGCGC (SEQ ID NO: 12) and
   - a set of primers targeting the CRISPR sequence SEQ ID NO: 3 wherein said primers are defined by the following sequences:
      GAACACTTTGGTGACAGTTTTTGT (SEQ ID NO: 13);
      CTTAGTGTGTTCCCCGCGC (SEQ ID NO: 14),
   wherein the presence of an amplification product for at least one of said sets of primers is indicative of the presence of EHEC O157:[H7]; and/or :
   - a set of primers targeting the CRISPR sequence SEQ ID NO: 4, wherein said primers are defined by the following sequences:
      GAACACAAACCGAAACACACG (SEQ ID NO: 15)
      ATAAACCGTCACCAAAACAGTG (SEQ ID NO: 16),
      wherein the presence of an amplification product for said set of primers is indicative of the presence of EHEC O157:[H7]; and
b) primers for detecting EHEC O145:[H28], wherein said primers consist of:
   - a set of primers targeting the CRISPR sequence SEQ ID NO: 5, wherein said primers are defined by the following sequences:
      GAACTTGAGCCCTGCCAGAA (SEQ ID NO: 17)
      ACCGCGATCTTTTCCTACCTG (SEQ ID NO: 18),
      wherein the presence of an amplification product for said set of primers is indicative of the presence of EHEC O145:[H28]; and
c) primers for detecting EHEC O111: [H8], wherein said primers consist of:
   - a set of primers targeting the CRISPR sequence SEQ ID NO: 6, wherein said primers are defined by the following sequences:
      GTGACCGCCTGTACACGC (SEQ ID NO: 19)
      CGGATATTTGGGCGTAATACC (SEQ ID NO: 20)
      CTGCCGCGAGTGGTTTCAC (SEQ ID NO: 21),
      wherein the presence of an amplification product for at least one of primers pairs SEQ ID NO: 19 and SEQ ID NO: 20 or SEQ ID NO: 19 and SEQ ID NO: 21 is indicative of the presence of EHEC O111:[H8]; and
d) primers for detecting EHEC O121:[H19], wherein said primers consist of:
   - a set of primers targeting the CRISPR sequence SEQ ID NO: 7, wherein said primers are defined by the following sequences:
      CGGGGAACACTACAGGAAAGAA (SEQ ID NO: 22)
      GGCGGAATACAGGACGGGTGG (SEQ ID NO: 23),
   wherein the presence of of an amplification product for said set of primers is indicative of the presence of EHEC O121: [H19]; and
e) primers for detecting EHEC O103:[H2] and/or EHEC O45:[H2], wherein said primers consist of:
   - a set of primers targeting the CRISPR sequence SEQ ID NO: 8, wherein said primers are defined by the following sequences:
      GAGTCTATCAGCGACACTACC (SEQ ID NO: 24)
      AACCGCAGCTCGCAGCGC (SEQ ID NO: 25),
   wherein the presence of an amplification product for said set of primers is indicative of the presence of EHEC O103:[H2] and/or of EHEC O45:[H2]; and
f) primers for detecting EHEC O104:[H4], wherein said primers consist of:
   - a set of primers targeting the CRISPR sequence SEQ ID NO: 9, wherein said primers are defined by the following sequences:
      GGAACTCACCGAGCGCCG (SEQ ID NO: 26);
      GCCTTTGCAGCGTCTTTCCGATC (SEQ ID NO: 27);
   wherein the presence of an amplification product for said set of primers is indicative of the presence of EHEC O104:[H4]; and
g) primers for detecting EHEC O26:[H11], wherein said primers consist of:
   - two sets of primers targeting the CRISPR sequence SEQ ID NO: 10, wherein the first primers set is defined by the following sequences:
      ACAATCGTGTGTAAATTCGCGG (SEQ ID NO: 28)
      GATAAACCGTGGTACGGAACA (SEQ ID NO: 29) and the second said primers set is defined by the following sequences:
      TGAAACCACTCGCGGCAGAT (SEQ ID NO: 30);
      ATAAACCGATCTCCTCATCCTC (SEQ ID NO: 31);
   wherein the presence of an amplification product for at least one of the said sets of primers is indicative of the presence of EHEC O26:[H11].

The amplification products can be detected by any appropriate method for detection of PCR products. For instance, they can be detected by means of probes derived from the respective target sequences.

Examples of preferred probes are given below:
- a probe allowing the detection of amplification products derived from SEQ ID NO: 1 and SEQ ID NO: 2, defined by the following sequence: CGATCAATCCGAATATGAGCGGT (SEQ ID NO: 32), and a probe allowing the detection of amplification products derived from SEQ ID NO: 3, defined by the following sequence: CACTGTTTTGGTGACGGTTTATCC (SEQ ID NO: 33), and/or a probe allowing the detection of amplification products derived from SEQ ID NO: 4, defined by the following sequence: ACAAAAACTGTCACCAAAGTGTTC (SEQ ID NO: 34);
- a probe allowing the detection of amplification products derived from SEQ ID NO: 5, defined by the following sequence:
   TGGGGCCTCTTTTGTACCCGG (SEQ ID NO: 35);
- a probe allowing the detection of amplification products derived from SEQ ID NO: 6, defined by the following sequence:
   TGTAATGGCTCACCGGTTTATCCCC (SEQ ID NO: 36);
- a probe allowing the detection of amplification products derived from SEQ ID NO: 7, defined by the following sequence:
   TCCGCCAACGGCGACAGGGG (SEQ ID NO: 37);
- a probe allowing the detection of amplification products derived from SEQ ID NO: 8, defined by the following sequence:
   TCGGAACGTGGCGCTATAGGTG (SEQ ID NO: 38);
- a probe allowing the detection of amplification products derived from SEQ ID NO: 9, defined by the following sequence:
   CTGGGAGGCGTATCTCACGTTCGGT (SEQ ID NO: 39);
- a probe allowing the detection of amplification products derived from SEQ ID NO: 10, defined by the following sequence:
   TGCTGTCTATATTTCGACCAGTGTTCC (SEQ ID NO: 40);
- a probe allowing the detection of amplification products derived from SEQ ID NO: 10, defined by the following sequence:
   CCAGCTACCGACAGTAGTGTGTTCC (SEQ ID NO: 41);

According to another aspect of the present invention, it provides a method for predicting whether a sample contains enterohemorrhagic *Escherichia coli* (EHEC) of at least one of EHEC O157:H7, O145:H28, O103:H2, O111:H8, O121:H19, O26:H11 and 045:H2 serotypes and their non-motile derivatives.

Said method comprises the detection of at least one of the following combinations of target genes:
- *Z2098* and at least one of *espK* and *espN;*
- *espN* and *espM1;*
- *espK* and *espV.*

*EspK, espM1, espN* and *espV* correspond to non LEE-encoded type III effectors derived from various genomic islands; *Z2098* is an ORF encoding an hypothetical protein of unknown function, present in the O-island 57.

The above combinations of genes were identified by the inventors among several combinations of putative virulence markers, as being the more predictive of the presence of at least one EHEC strain of serotypes EHEC O157:[H7], O145:[H28], O103:[H2], O111:[H8], O121:[H19], O26:[H11] or O45:[H2].

More specifically said method comprises performing a PCR assay on said sample or DNA isolated therefrom with a combination of primers selected among:
- a combination of primers comprising a set of primers derived from *Z2098* and a set of primers derived from at least one of *espK* and *espN;*
- a combination of primers comprising a set of primers derived from *espN* and a set of primers derived from *espM1;*
- a combination of primers comprising a set of primers derived from *espK* and a set of primers derived from *espV;*
and detecting the presence or the absence of an amplification product for each set of primers of said combination.

According to a preferred embodiment of this method, the combination of primers further comprises a set of primers derived from *stx1* and a set of primers derived from *stx2*.This allows screening samples for both the *stx* genes, as markers of STEC, and for the addititional genetic markers listed above, related to priority STEC serotypes that are associated with outbreaks and sporadic cases of HC and HUS.

In contrast to the prior art methods, the method of the invention does not necessitate the detection of the *eae* gene.

Primers derived from *Z2098, espK, espN, espM1, espV stx1* and *stx2* and suitable for use in the PCR assay of the invention, as well as probes allowing the detection of the amplification products obtained with these primers, can easily be designed by one of skill in the art, on the basis of the sequences of these genes available in the databases, for instance within the annotated sequence of *Escherichia coli* O157:H7 (strain EDL933) available in GenBank under accession number AE005174.2 .

Non-limitative examples of preferred sets of primers for use in this PCR assay are given below:
- a set of primers targeting Z2098, defined by the following sequences:
   CTGAAAAGAGCCAGAACGTGC (SEQ ID NO: 42)
   TGCCTAAGATCATTACCCGGAC (SEQ ID NO: 43)
- a set of primers targeting *espK,* defined by the following sequences:
   GCAGRCATCAAAAGCGAAATCACACC (SEQ ID NO: 44)
   TCGTTTGGTAACTGTGGCAGATACTC (SEQ ID NO: 45)
- a set of primers targeting *espN,* defined by the following sequences:
   GACATATTTGTTTATGTCATCAGGAGCGG (SEQ ID NO: 46)
   CCTCAGGATATGGATGGCCTACTGGC (SEQ ID NO: 47)
- a set of primers targeting *espM1,* defined by the following sequences:
   GCGCTCTATCCGCTTTAATGTTAAC (SEQ ID NO: 48)
   CCATCCATGAATATCTTTAGTACTCTGC (SEQ ID NO: 49)
- a set of primers targeting *espV,* defined by the following sequences:
   TCAGGTTCCTCGTCTGATGCCGC (SEQ ID NO: 50)
   CTGGTTCAGGCCTGGAGCAGTCC (SEQ ID NO: 51)
- a set of primers targeting *stx1* and *stx2,* defined by the following sequences:
   TTTGTYACTGTSACAGCWGAAGCYTTACG (SEQ ID NO: 52)
   CCCCAGTTCARWGTRAGRTCMACRTC (SEQ ID NO: 53)

Non-limitative examples of probes for detecting the amplification products are given bellow:
- a probe allowing the detection of amplification products derived from Z2098, defined by the following sequence:
   TAACTGCTATACCTCCGCGCCG (SEQ ID NO: 54);
- a probe allowing the detection of amplification products derived from *espK,* defined by the following sequence:
   ATTCAGATAGAAGAAGCGCGGGCCAG (SEQ ID NO: 55);
- a probe allowing the detection of amplification products derived from *espN,* defined by the following sequence:
   AATGCTCTCGGCAATCGAATCCTTGACTC (SEQ ID NO: 56);
- a probe allowing the detection of amplification products derived from *espM1*, defined by the following sequence:
   TGCTTACCGTCTCCAGTATACAGCCGCT (SEQ ID NO: 57);
- a probe allowing the detection of amplification products derived from *espV,* defined by the following sequence:
   CTTGCAACACGTTACGCTGCCGAGTATT (SEQ ID NO: 58);
- a probe allowing the detection of amplification products derived from *stx1,* defined by the following sequence:
   CTGGATGATCTCAGTGGGCGTTCTTATGTAA (SEQ ID NO: 59);
- a probe allowing the detection of amplification products derived from *stx2,* defined by the following sequence:
   TCGTCAGGCACTGTCTGAAACTGCTCC (SEQ ID NO: 60);

Advantageously, the invention provides a method for predicting whether a sample contains enterohemorrhagic *Escherichia coli* (EHEC) of at least one of EHEC O157:[H7], O145:[H28], O103:[H2], O111:[H8], O121:[H19], O26:[H11] and O45:[H2] serotypes, and further identifying the serotype(s) of said EHEC, wherein said method comprises:
- performing a PCR assay for assessing whether or not said sample comprises EHEC of at least one of O157:[H7], O145:[H28], O103:[H2], O111:[H8], O121:[H19], O26:[H11] and O45:[H2] serotypes, as described above, and if the results of said PCR assay are positive,
- performing a PCR assay for identifying the serotype(s) of said EHEC, as described above.

The PCR assays of the invention can be used for testing any sample of a substance potentially containing EHEC, such as food samples, water samples, soil samples, etc.

The PCR assays of the invention can be carried out using any method suitable for PCR amplification of target sequences, using any of the various natural or engineered enzymes available for this purpose. Alternative methods such as nucleic acid sequence-based amplification (NASBA), branched DNA, strand displacement amplification or the loop-mediated isothermal amplification (LAMP) method (Compton 1991, Chang 1991, Walker et al.1992, Notomi et al., 2000) can also be used.

Particularly preferred methods are those involving real time PCR amplification as described by Ian M. Mackay in "Real-time PCR in Microbiology : from diagnosis to characterization" (2007) Caister Academic Press, Norfolk, UK.

Real time PCR, also called quantitative real time polymerase chain reaction (qPCR) or kinetic polymerase chain reaction, is used to amplify and simultaneously quantify a targeted DNA molecule. It enables both detection and quantification (as absolute number of copies or relative amount when normalized to DNA input or additional normalizing genes) of a specific sequence in a DNA sample. The procedure follows the general principle of polymerase chain reaction; its key feature is that the amplified DNA is quantified as it accumulates in the reaction in real time after each amplification cycle (Mackay 2007). Two common methods of quantification are the use of fluorescent dyes that intercalate with double-strand DNA, and modified DNA oligonucleotide probes that fluoresce when hybridized with a complementary DNA (Mackay 2007). In the present invention the inventors have shown the second of these two methods, but the other method of quantifying PCR products based upon intercalating fluorescent dyes is also within the scope of the present invention.

Non-limiting examples of suitable fluorescent labels include 6-carboxylfluorescein (FAM), tetrachloro-6-carboxyfluorescein (TET), 6-carboxy-X-rhodamine (ROX). Non-limitative examples of suitable quenchers for labelling dual-labelled probes include 6-carboxy-tetramethyl-rhodamine (TAMRA), DABCYL, Non-Fluorescent Quenchers such as quenchers of the Black Hole Quencher family (BHQ), or including a minor groove binder group (MGB).

Each of the PCR assays of the invention can be carried out by performing a separate PCR reaction for each target sequence to be detected (simplex PCR). However, in many cases it will be preferred to carry out multiplex PCR, allowing amplification of several target sequences in a single reaction. Advantageously, one can use a macroarray, i.e. a preformed structure such as a substrate upon which the desired DNA primers have been spotted. Such a macroarray allows the routine performance of multiplex PCR assays described herein. By way of example, one can use the GeneDisc® macroarray (Pall-GeneDisc Technology, Bruz, France) described for instance by Beutin et al. (Beutin et al.2009) which allows the simultaneous detection of multiple targets in reaction microchambers preloaded with the reagents necessary for detecting and quantifying the required targets.

In order to ensure that the results of the assay are representative of the true contents of the sample, it may also comprise a negative amplification control to ensure any detected products are true positives and also an inhibition control to ensure that the DNA from the sample is able to be amplified and hence that no false negatives are generated.

The invention also encompasses the primer sets and the probes defined above, allowing to carry out the PCR assays of the invention, as well as kits associating these primer sets and these probes, eventually associated with reagents to perform a PCR reaction. These kits may also comprise instructions for performing said amplification reaction. The amplification products using the primers of the invention, are also part of the invention.

According to a first embodiment, a kit of the invention comprises a combination of primers comprising:
- a set of primers defined by the sequences SEQ ID NO: 11 and SEQ ID NO: 12 and a set of primers defined by the sequences SEQ ID NO: 13 and SEQ ID NO: 14, and/or a set of primers defined by the sequences SEQ ID NO: 15 and SEQ ID NO: 16;
- a set of primers defined by the sequences SEQ ID NO: 17 and SEQ ID NO: 18;
- a set of primers defined by the sequences SEQ ID NO: 19, SEQ ID NO: 20, and SEQ ID NO: 21;
- a set of primers defined by the sequences SEQ ID NO: 22 and SEQ ID NO: 23;
- a set of primers defined by the sequences SEQ ID NO: 24 and SEQ ID NO: 25;
- a set of primers defined by the sequences SEQ ID NO: 26 and SEQ ID NO: 27;
- a set of primers defined by the sequences SEQ ID NO: 28 and SEQ ID NO: 29;
- a set of primers defined by the sequences SEQ ID NO: 30 and SEQ ID NO: 31;

Preferably, said kit also comprises:
- a probe allowing the detection of amplification products derived from SEQ ID NO: 1 and SEQ ID NO: 2, and a probe allowing the detection of amplification products derived from SEQ ID NO: 3, and/or a probe allowing the detection of amplification products derived from SEQ ID NO: 4, as defined above;
- a probe allowing the detection of amplification products derived from SEQ ID NO: 5, as defined above;
- a probe allowing the detection of amplification products derived from SEQ ID NO: 6, as defined above;
- a probe allowing the detection of amplification products derived from SEQ ID NO: 7, as defined above;
- a probe allowing the detection of amplification products derived from SEQ ID NO: 8, as defined above;
- a probe allowing the detection of amplification products derived from SEQ ID NO: 9, as defined above;
- two probes allowing the detection of amplification products derived from SEQ ID NO: 10, as defined above.

According to a second embodiment, a kit of the invention comprises:
- a set of primers derived from *Z2098,* and
- a set of primers derived from *espK* or a set of primers derived from *espN.*

Preferably, said kit also comprises a probe allowing the detection of amplification products derived from Z2098, and a probe allowing the detection of amplification products derived from *espK,* or a probe allowing the detection of amplification products derived from *espN.*

According to a third embodiment, a kit of the invention comprises:
- a set of primers derived from *espN* and a set of primers derived from *espM1.*

Preferably, said kit also comprises a probe allowing the detection of amplification products derived from *espN,* and a probe allowing the detection of amplification products derived from *espM1.*

According to a fourth embodiment, a kit of the invention comprises:
- a set of primers derived from *espK* and a set of primers derived from *espV.*

Preferably, said kit also comprises a probe allowing the detection of amplification products derived from *espK,* and a probe allowing the detection of amplification products derived from *espV.*

The kits according to the second, third and fourth embodiment described above may further comprise a set of primers targeting *stx1* and a set of primers targeting *stx2,* and preferably a probe allowing the detection of amplification products derived from *stx1,* and a probe allowing the detection of amplification products derived from *stx2.*

For a better understanding of the invention and to show how the same may be carried into effect, there will now be shown by way of example only, specific embodiments, methods and processes according to the present invention.

### EXAMPLE 1: IDENTIFICATION OF DNA SEQUENCES DERIVED FROM THE CRISPRS LOCI OF E. COLI FOR SPECIFIC IDENTIFICATION OF ENTEROHAEMORRHAGIC E. COLI (EHEC)

### Materials and methods

### Bacterial strains

Strains of *E. coli* (n = 955) that were investigated for their CRISPR loci by high throughput real-time PCR are reported in Table I below.

**Table I: E. coli strains**

| **EHEC*** (n = 331) |
|---|
| O103:[H25] (n=6), **O103:H2 (n=38), O111:H8 (n=49),** O118:[H16] (n=3), O119:[H25] (n=4), **O121:H19 (n=12),** O123:H11, O127:H8s, O145, **O145:[H28] (n=29),** O156:H21, O156:H25 (n=10), **O157:[H7] (n=75),** O165:H25, O172:[H25], O172:H25, O172:NM, O177 (n=2), O177:[H25], O182:[H25], **O26:[H11] (n=76),** O3, **O45:H2,** O49:H16, O5 (n=8), O55, O76:H51, O84:H2, Ont:[H2], Or:H16, OX186:[H2] |

| **EPEC** (n = 344) |
|---|
| O100:[H25] (n=2), O102:H19, O103:H21, O103:H8, O108:H9 (n=6), O109:H25, O111, O111:B4, O111:H11, O111:H19 (n=3), O111:H2 (n=13), O111:H25 (n=2), O111:H47, O111:H9 (n=3), O113:H6 (n=2), O114:H2 (n=6), O114:H49 (n=5), O115:H38 (n=3), O117:H25, O117:H40b (n=3), O118:H5, O118:H8a (n=3), O119:[H25], O119:H2 (n=3), O119:H6 (n=4), O119:H8 (n=2), O119:H9, O119s:H2, O123/o4:H45 (n=2), O123:H25, O125:H6, O125ac:H6 (n=3), O126:H27, O126:H6, O127, O127:H19, O127:H40 (n=4), O127:H40b (n=2), O127:H6 (n=2), O128:[H2] (n=12), O128:H8, O128ac:H2, O142:H34, O142:H6 (n=3), O145:H34 (n=5), O15:H11, O15:H2 (n=3), O153:H14, O156, O156:[H8] (n=7), O156:H1 (n=2), O156:H25 (n=3), O157, O157:[H45] (n=2), O157:H16 (n=5), O157:H2, O157:H26 (n=2), O157:H39, O157:H45 (n=3), O177:H26, O186:[H45], O2:[H40] (n=2), O2:H40b, O2:H8, O21:H25, O22:H7, O26:[H11] (n=38), O26:H31, O26:H34, O28:H28 (n=4), O3:H40b, O3:H5, O3:H8a (n=3), O37:H10, O4:H16, O45, O45:H7, O45:H9, O49:[H10] (n=2), O49:H-, O5:H11, O51:H49 (n=3), O55:[H51], O55:[H7] (n=26), O55:H6 (n=5), O62:H9, O63:H6 (n=2), O66:H8/8a, O69:[H2], O69:H16 (n=2), O70/O86:H2, O70:H11 (n=5), O71:H40b, O76:H41, O76:H7 (n=5), O80:[H2] (n=3), O84:[H2], O86:[H34] (n=4), O86:H11 (n=2), O86:H40, O86:H8 (n=4), O86:H8a, O88:H8a, O89:[H2], O9:H10, OK8:H10, Ont:[H10], Ont:[H6], Ont:H11, Ont:H14, Ont:H2 (n=2), Ont:H21, Or:H40b, Or:H8a, Or:H9, OX177:H11 (n=2), OX177:H6 |

| **STEC**** (n = 160) |
|---|
| O100:NM (n=2), O101:H- (n=3), O104:H7, O105:H18, O109:H-, O110:H28, O111, O111:H10, O113:H4, O115:H18 (n=2), O116:H28, O117 (n=2), O117:H7 (n=2), O118:H12 (n=3), O125, O126, O126:H8, O128ab:H2, O130:H11, O136 (n=2), O138, O139, O139:H1, O141:[H4], O141ac, O146:H21, O146:H28 (n=2), O146:H8, O147, O149:H19, O15:H16, O153:H25 (n=3), O165:H11, O168:H8, O17/77:H41, O171:H-, O171:H2, O172:H21, O174:[H21] (n=11), O174:H2, O174:H8 (n=4), O176:H-, O178:H19, O179:H8, O181:H49, O2:H25, O2:H27, O21:H21 (n=3), O22/083, O22:H16 (n=2), O22:H8 (n=3), O23:H15, O3, O39:H48, O40:21, O40:H8, O46:H38 (n=2), O48:H21, O5, O5:[H19], O53, O6 (n=7), O6:H10 (n=2), O6:H34 (n=2), O68:H12, O73:H18, O74:H42, O75:H8, O76, O76:H19 (n=3), O77 (n=2), O79, O79:H48, O8:H10, O8:H19 (n=6), O8:H8, O85:H11, O86, O88:H25, O91 (n=6), O91:[H21] (n=5), O91:H10 (n=3), O91:H14 (n=2), O92,O107:H-, O92,O107:H48, O96:H19, Ont:H-, Ont:H7, Or:[H16], Or:H12, Or:H29, Or:H33, Or:H4, Or:H48, OX178:H19, OX185:H28, OX187:Hbev, OX3:H-, OX3:H2, OX3:H21, OX7:H16 |

| **Apathogenic *E. coli*** (n = 120) |
|---|
| O103 (n=2), O103:H8, O104:H7, O110, O111:H12, O111:H21, O121:[H45], O126 (n=33), O126:H11, O126:H27 (n=3), O127 (n=8), O127:H10, O127:H21, O142 (n=8), O145:H2 (n=2), O150:H8, O153:H12, O156:H33, O156:H47, O156:H56, O157 (n=5), O157:H27, O180:H-, 026:H? (n=4), O26:H21/32, O26:H32 (n=6), O26:NM, O4:H5, O41:H7, O45:H7, O55 (n=8), O55:H19, O55:H21, O6:H4, O62:H30 (n=2), O8/O104:H10, O8/O104:H45, O86 (n=6), O86/O125ac, O86:H2, O86:H27, O88, O9:K9:H12, OX183:H18 |

| |
|---|
| For each serotype, n=1 unless otherwise stated. * Including EHEC derivatives as described in (Bugarel et al. 2010). EHEC from the top 7 serotypes (Typical EHEC) are noted in bold. ** Including atypical EHEC. |

*E. coli* strains were divided into Shiga-toxin producing *E. coli* or STEC (n = 160), enteropathogenic *E. coli* or EPEC (n = 344), enterohaemorrhagic *E. coli* or EHEC (n = 331) and apathogenic *E. coli* (n = 120). The STEC/EHEC type was defined on the presence of *stx-* and *eae*-genes. EHEC strains were defined as harbouring both a *stx* gene (*stx1* and/or *stx2*) and *eae,* while STEC strains harboured *stx* only. STEC included *stx*-positive and *eae-*negative *E. coli* strains of serotypes O91:[H21], O113:[H21], O104:[H21], also named atypical EHEC, which are less frequently involved in hemorrhagic diseases than other EHEC, but are a frequent cause of diarrhea. *Stx*-negative derivatives of EHEC strains were designated as EHEC-like and were defined based on their *nle* gene profile, *eae* subtype and serotype as described by Bugarel et al. (2010; 2011) except for the EHEC-like strains of serotype O26:H11 which were identified based on the presence of the gene *espK* and their allelic type 2 of the *arcA* gene (Bugarel et al., 2011). EPEC strains were defined as described by Bugarel et al. (2011). Apathogenic *E. coli* were defined as *stx-* and *eae*- negative strains.

All strains investigated in this work were identified for the *E. coli* 0 (LPS) and H (flagellar) antigens and have been characterized for the *stx-* and *eae-* genes as previously reported (Bugarel et al. 2010). For examination, bacteria were cultured to single colonies on Luria-Broth Plates and grown overnight at 37°C. One colony was picked-up and DNA extracted using the InstaGene matrix (Bio-Rad Laboratories, Marnes La Coquette, France) before high throughput real-time PCR testing.

### DNA Sequencing

The CRISPR loci of E. *coli* strains were PCR amplified with the primers listed in Table II. The double stranded DNA sequencing of the CRISPR amplicons was performed by Eurofins MWG Operon (Courtaboeuf, France) using the sequencing primers listed in Table II.

**Table II**

| Primer name | Forward primer and reverse primer sequences (5' - 3') | SEQ ID | Accession | Location within sequence |
|---|---|---|---|---|
| | | NO: | Number | |
| | | | | |
| CRISPR-I-F | GGTGAAGGAGYTGGCGAAGGCGTC | 61 | AE005174 | 3665412-3665435 |
| CRISPR-I-R | CCGGTGGATTTGGATGGGTTACG | 62 | AE005174 | 3665885-3665863 |
| CRISPR-II-F | TGTGAACCTCTCTGGCATGGAG | 63 | AP010953 | 3786919-3786940 |
| CRISPR-II-R | TAAAGTTGGTAGATTGTGACTGGC | 64 | AP010953 | 3787672-3787649 |

### High-throughput real-time PCR

The LightCycler® 1536 (Roche, Meylan, France) was used to perform high-throughput real-time PCR amplifications. For the PCR setup of the LightCycler® 1536 multiwell plates, the Bravo liquid dispenser automat (Agilent Technologies, Massy, France) equipped with a chiller and the PlateLoc thermal microplate sealer (Agilent Technologies) were used. The PCR reactions contained 0.5 µl sample and 1 µl master mix containing 1x RealTime ready DNA Probes master (Roche) (corresponding to 0.7x final), 300 nM each primer and 300 nM each probe (corresponding to 200 nM final each). Amplifications were performed using FAM- or HEX-labeled TaqMan® probes. Primers and probes used for PCR amplifications are listed in Table III. The LightCycler® 1536 real-time PCR system was used with the following thermal profile: 95°C for 1 min followed by 35 cycles of 95°C for 0 s (ramp: 4.8°C/s) and 60°C for 30 s (ramp: 2.5°C/s) and a final cooling step at 40°C for 30s. The software settings were Dual color hydrolysis probes/UPL probes and Master Control.

**Table III**

| | Forward primer, reverse primer and probe sequences (5' -3') | SEQ ID NO: | Target sequence |
|---|---|---|---|
| SP_O157_A | GAACACAAACCGAAACACACG | 15 | (SEQ ID NO: 4) |
| | ATAAACCGTCACCAAAACAGTG | 16 | |
| | [FAM]-ACAAAAACTGTCACCAAAGTGTTC-[BHQ1] | 34 | |
| SP_O157_B | GGGAACACAAACCGAAACACA | 11 | (SEQ ID NO: 1 |
| | CTTAGTGTGTTCCCCGCGC | 12 | and 2) |
| | [HEX]-CGATCAATCCGAATATGAGCGGT-[BHQ1] | 32 | |
| SP_O157_C | GAACACTTTGGTGACAGTTTTTTGT | 13 | (SEQ ID NO: 3) |
| | CTTAGTGTGTTCCCCGCGC | 14 | |
| | [HEX]-CACTGTTTTGGTGACGGTTTATCC-[BHQ1] | 33 | |
| | | | |
| SP_O121 | CGGGGAACACTACAGGAAAGAA | 22 | (SEQ ID NO: 7) |
| | GGCGGAATACAGGACGGGTGG | 23 | |
| | [HEX]-TCCGCCAACGGCGACAGGGG-[BHQ1] | 37 | |
| SP_O45 | GAGTCTATCAGCGACACTACC | 24 | (SEQ ID NO: 8) |
| | AACCGCAGCTCGCAGCGC | 25 | |
| | [HEX]-TCGGAACGTGGCGCTATAGGTG-[BHQ1] | 38 | |
| SP_O145 | GAACTTGAGCCCTGCCAGAA | 17 | (SEQ ID NO: 5) |
| | ACCGCGATCTTTTCCTACCTG | 18 | |
| | [HEX]-TGGGGCCTCTTTTGTACCCGG-[BHQ1] | 35 | |
| SP_O104 | GGAACTCACCGAGCGCCG | 26 | (SEQ ID NO: 9) |
| | GCCTTTGCAGCGTCTTTCCGATC | 27 | |
| | [HEX]-CTGGGAGGCGTATCTCACGTTCGGT-[BHQ1] | 39 | |
| SP_O26_C | ACAATCGTGTGTAAATTCGCGG | 28 | (SEQ_ID_NO:10) |
| | GATAAACCGTGGTACGGAACA | 29 | |
| | [HEX]-TGCTGTCTATATTTCGACCAGTGTTCC-[BHQ1] | 40 | |
| SP_O26_D | TGAAACCACTCGCGGCAGAT | 30 | (SEQ ID NO:10) |
| | ATAAACCGATCTCCTCATCCTC | 31 | |
| | [HEX]-CCAGCTACCGACAGTAGTGTGTTCC-[BHQ1] | 41 | |
| SP_O111 | GTGACCGCCTGTACACGC | 19 | (SEQ ID NO: 6) |
| | CGGATATTTGGGCGTAATACC | 20 | |
| | CTGCCGCGAGTGGTTTCAC | 21 | |
| | [HEX]-TGTAATGGCTCACCGGTTTATCCCC-[BHQ1] | 36 | |

### Results

### Identification of specific DNA sequences targeting the CRISPRs loci of EHEC O157:H7

Sequencing the CRISPR loci of various EHEC O157:[H7] strains has shown the polymorphism of this locus for this serotype. Sequences characteristic of the CRISPR loci of EHEC O157:[H7] strains are reported in SEQ ID NO: 1, 2, 3 and 4. Based on these sequences and the CRISPR locus of the strain EDL933 (Accession number AE005174), various real-time PCR assays were designed (SP_O157_A, SP_O157_B and SP_O157_C) for detecting EHEC O157:[H7]. The specificity and sensitivity of the assays was tested against a panel of 955 E. *coli* strains, including 75 strains of EHEC O157:[H7] (Table I). The PCR tests proved to be highly sensitive and specific for EHEC O157:[H7]. Sensitivity of the assays was ranging from 92.0% to 97.3% with only few O157:[H7] strains being not detected by each assay. The specificity of the PCR tests was high, ranging from 99.6 to 100%. The PCR assay SP_O157_B was the unique test giving cross reaction with very few strains of serogroup 055. By combining the PCR assays SP_O157_B and SP_O157_C all the 75 EHEC O157:[H7] strains were correctly detected (100% sensitivity) and only 3 isolates of serogroup 055 were cross-reacting (99.6% specificity).

### Identification of specific DNA sequences targeting the CRISPR locus of EHEC O145:H28

The CRISPR locus of EHEC O145:[H28] has been characterized (SEQ ID NO: 5) by sequencing one of the two CRISPR loci identified in *E. coli.* A PCR assay (SP_O145) has been designed from this CRISPR sequence to target EHEC O145:[H28]. Among the 955 *E. coli* strains that were investigated with this PCR test, only the 29 EHEC O145:[H28] and 4 EPEC O28:H28 strains were tested positive. Sensitivity and specificity of the PCR assay SP_O145 were respectively of 100% and 99.5%.

### Identification of specific DNA sequences targeting the CRISPR locus of EHEC O111:H8

Based on the sequence of the CRISPR locus of EHEC O111:H8, (SEQ ID NO: 6), a real-time PCR assay has been designed (SP_O111) to detect EHEC O111:[H8]. Investigation of 980 *E. coli* strains by the PCR assay SP_O111 gave positive results for 47 EHEC O111: [H8] out of the 49 O111: [H8] strains tested. Only one EPEC strain of serotype O45:H7 was tested positive. Sensitivity and specificity of this PCR assay were high, 95.9% and 99.9% respectively.

### Identification of specific DNA sequences targeting the CRISPR locus of EHEC O121:H19

The CRISPR locus of EHEC O121:[H19] has been sequenced in this study (SEQ ID NO: 7). A PCR assay (SP_O121) has been designed from this sequence to target EHEC O121:[H19]. Among the 955 *E. coli* strains tested by the PCR assay SP_O121, only one O104:H7 and the 12 EHEC O121:[H19] strains were tested positive, showing that this PCR test was highly sensitive (100%) and specific (99.9%).

### Identification of specific DNA sequences targeting the CRISPRs loci of EHEC O103:H2 and O45:H2

Based on the sequence determination of the CRISPR locus of EHEC O45:[H2] (SEQ ID NO: 8) and the sequence of the CRISPR locus of EHEC O103:H2, issued from strain 12009 (accession number AP010958), a PCR assay (SP_O45) has been designed and tested positive one strain of EHEC O45:H2 and all the 38 EHEC 0103:H2 strains investigated in this study. Thus, the PCR assay SP_O45 has shown high sensitivity (100%) for EHEC O103:[H2] and O45:[H2]. This test has 98.6% specificity when tested on a large panel of *E. coli,* giving only minor cross-reactions with few strains of the following serotypes: O118:H8, O128:[H2], O128:H8, O128:H2, O89:[H2], O46:H38, O8:H8, O142, O145:H2 and one O103 strain that tested negative for the flagella H2.

### Identification of specific DNA sequences targeting the CRISPR locus of EHEC O104:H4

The CRISPR locus of EHEC O104:[H4] has been sequenced in this study (SEQ ID NO: 9). A PCR assay (SP_O104) has been designed from this sequence to target EHEC O104:[H4]. The PCR assay targeting the CRISPR locus of *E. coli* 0104:H4 has been evaluated on a panel of 1303 strains of *E*. *coli* that included the 186 known O-serogroups and 56 H-types. This PCR assay gave positive results for the 48 O104:H4 isolates (including one Or:H4 isolate) related to the outbreak occurring in May 2011, and to one O104:H4 clinical isolate reported in 2001. The 39 strains of *E. coli* O104 having other H-types than H4 were tested negative. The *E. coli* strains carrying a K9 capsular antigen (O8:K9:H10, O8:K9:H45, O9:K9:H1, O9:K9:H12 and O9:K9:H51) which cross react by agglutination with the sera anti-O104 tested all negative. In final, among the other *E. coli* strains that included the 186 known O-serogroups and 56 H-types, only 5 isolates belonging to serotypes Ont :H2, O43:H2, O141:H2, and O174:H2 were cross reacting with the primers and probes designed in the CRISPR locus of EHEC O104:H4. Additional O174:H2, O141:H2 and 043:H2 strains from the BfR strains collection were thus tested for CRISPR-O104. Three out of twelve O174:H2 tested positive, as well as 3/4 O43:H2 and 1/8 O141:H2. All together the data showed that that this PCR test was highly sensitive (100%) and specific (99.6%).

### Identification of specific DNA sequences targeting the CRISPR locus of EHEC O26:H11

Sequencing the CRISPR loci of various EHEC O26:[H11] strains has shown the polymorphism of this locus for this serotype. A Sequence characteristic of the CRISPR loci of EHEC O26:[H11] is reported in SEQ ID NO: 10. Based on these sequences and the CRISPR locus of the EHEC O26:H11 strain 11368 (Accession numbers AP010953, NC_013361), two real-time PCR assays were designed (SP_O26_C, and SP_O26_D) for detecting EHEC O26:[H11]. The specificity and sensitivity of the assays was tested against a panel of 980 *E. coli* strains, including 77 strains of EHEC O26:[H11] and EHEC-like O26:[H11]. The two PCR tests proved to be sensitive and specific for EHEC O26:[H11]. Sensitivity of the SP_O26_C PCR assay was 87.0% whereas the sensitivity of SP_O26_D PCR assay was 90.9%. Only few O26:[H11] strains were not detected by each assay. The specificity of the PCR test SP_O26_C was 98.7% (12 strains cross-reacting) whereas the specificity of the PCR test SP_O26_D was 98.1% (17 strains cross-reacting). By combining the PCR assays SP_O26_C and SP_O26_D only 4 EHEC-like O26:H11 strains out of the 77 EHEC-like and EHEC O26:[H11] strains were not detected (94.8% sensitivity) and only 26 *E. coli* were cross-reacting (97.1% specificity).

### Conclusion

The results of this study are summarized in Table IV below.

**Table IV: Sensitivity and specificity**

| Serotype | Number | PCR | Sensitivity | Specificity | Cross-reaction |
|---|---|---|---|---|---|
| | | SP_O157_A | 92.0% | 100% | - |
| O157:[H7]^{a} | 75 | SP_O157_B | 97.3% | 99.6% | O55:[H7]^{a}, O55:[H7] (n=2)^{b} |
| | | SP_O157_C | 94.7% | 100% | - |
| | | SP_O157_B+C | 100% | 99.6% | O55:[H7]^{a}, O55:[H7] (n=2)^{b} |
| O103:H2^{a}, O45:H2^{a} | 38 1 | SP_O45 | 100% | 98.6% | O118:H8a (n=3)^{b}, O128:[H2]^{b}, O128:H8^{b}, O128ac;H2^{b}, O89:[H2]^{b}, O46:H38^{c}, O8:H8^{c}, O103^{d}, O142^{d}, O145:H2^{d} |
| O111:H8^{a} | 49 | SP_O111 | 95.9% | 99.9% | O45:H7 (n=1)^{b} |
| O121:H19^{a} | 12 | SP_O121 | 100% | 99.9% | O104:H7^{d} |
| O145:[H28]^{a} | 29 | SP_O145 | 100% | 99.5% | 028:H28 (n=4)^{b} |
| O104:[H4]^{a} | 49 | SP_O104 | 100% | 99.6% (n=2), and O174:H2 (n=4) | Ont :H2, O43:H2 (n=4), O141:H2 |
| O26:[H11]^{a} | 77 | SP_O26_C | 87% | 98.7% | O111:H11^{b}, O111:H47^{b}, O118:H16(n=2)^{a}, O118:H8a (n=3)^{b}, O128:H8^{b}, O26:H11^{b} O118:H2^{a}, O103:H11^{a}, O111^{b} |
| | | SP_O26_D | 90.9% | 98.1% | O118:H16 (n=3)^{a}, O123:H11^{a}, O26:H11 (n=9)^{b}, O118:H2 (n=2)^{a}, O86:H11 (n=2)^{b}, O103:H11^{a} |
| | | SP_O26_C+D | 94.8% | 97.1% | O111:H11^{b}, O111:H47^{b}, O118:H16 (n=4)^{a}, O118:H8a (n=3)^{b}, O123:H11^{a}, O128:H8^{b}, O26:H11 (n=10)^{b}, O86:H11 (n=2)^{b}, O118:H2 (n=2)^{a}, O103:H11^{a}, O111^{b} |

| | | | | | |
|---|---|---|---|---|---|
| For each serotype, n=1 unless otherwise stated. ^{a)}EHEC & EHEC-like; ^{b)}EPEC; ^{c)}STEC & atypical EHEC; ^{d)}non pathogenic *E*. *coli* | | | | | |

Sequencing the CRISPR loci of various EHEC strains has shown the genetic diversity of the CRISPR sequences issued from EHEC associated with the world's most frequent clinical cases. Analysis of the spacer sequences located between the short palindromic repeat sequences of the CRISPR loci, allowed identifying useful genetic markers to detect with high sensitivity and specificity EHEC strains. Based on a high-throughput real-time PCR approach, a very large panel of *E. coli* strains, that comprised EHEC, EPEC, STEC and apathogenic *E. coli* was investigated with regards to their CRISPR loci content. In final, EHEC O145:H28 (n=29), O103:H2 (n=38), O121:H19 (n=12), O104:H4 (n=49) and 045:H2 (n=1) were detected with 100% sensitivity with each PCR assays targeting various CRISPR sequences derived from these EHEC serotypes. EHEC O157:[H7] (n=75) was detected with 100% sensitivity when combining the PCR assays SP_O157_B and SP_O157_C which target two different sequences of the EHEC O157 CRISPR loci. EHEC O111:[H8] (n=49) was detected with 95.9% sensitivity (47/49 O111:[H8] were detected, only two were not detected). When combining the PCR assays SP_O26_C and SP_O26_D which target two different sequences of the O26 CRISPR loci, EHEC O26:[H11] (n=77) was detected with 94.8% sensitivity (73/77 O26:[H11] were detected; the only 4 strains which are not detected were EHEC-like O26:H11 strains)

The PCR assays developed in this study for targeting the CRISPR loci of EHEC associated with the world's most frequent clinical cases were also highly specific. These assays had 97.1% to 100% specificity when tested on a very large panel of *E. coli* strains, giving only very minor cross-reactions (Table IV).

### EXAMPLE 2: IDENTIFICATION OF GENETIC MARKERS FOR IDENTIFYING SHIGA TOXIN-PRODUCING ESCHERICHIA COLI (STEC) ASSOCIATED WITH HIGH VIRULENCE FOR HUMANS

### 1) Genetic markers ecs1763, espM1, espM2, espN, espO1-1, espW, espX7, terE and iha

The extended repertoire of non-LEE-encoded type III effectors (Tobe et al., 2006; Creuzburg et al., 2011) and adhesins (Spears et al., 2006; Cergole-Novella et al., 2007;) represents a most probable source of STEC virulence determinants.

The distribution of nine genetic markers, mostly non-LEE-encoded type III effectors, among various *E. coli* pathogroups was examined to assess their association with STEC strains with high virulence for humans.

### Materials and methods

The 696 *E. coli* strains investigated in this study were divided into enterohaemorrhagic *E. coli* or EHEC (n = 327, including 167 EHEC O26:H11), enteropathogenic *E. coli* or EPEC (n = 138, including 81 EPEC O26:H11), Shiga-toxin producing *E. coli* or STEC (n = 180) and apathogenic *E. coli* (n = 51), using the criteria indicated in Example 1.

These strains are listed in Table V below:

**Table V:**

| **EHEC** * (n = 327) |
|---|
| **O103:H2 (n=18),** O103:H25 (n=2), **O111:H8 (n=19),** O118:[H16] (n=3), O118:H+, **O121:H19 (n=8),** O123:H11, **O145:H28 (n=16),** O15:H2, O156:H25 (n=10), **O157:H7 (n=62),** O165:H25, O172:[H25], O177 (n=2), **O26:H11 (n=167), O45:H2 (n=2),** O49:H16, O5 (n=8), O55, O55:H7, Or:H16, OX186:[H2] |

| **EPEC** (n = 138) |
|---|
| O103:H21, O103:H8, O111:H11, O111:H2, O111:H25, O111:H47, O113 (n=3), O113:H6, O121, O125:H6, O126:H6, O127:H6, O128:H2 (n=3), O128ac:H2, O15:H2, O156, O156:H1 (n=2), O156:H25 (n=3), O156:H8 (n=6), O157, O157:H16 (n=2), O157:H2, O157:H26, O157:H39, O157:H45 (n=2), O26:H11 (n=81), O26:H31, O26:H34, O55:H6, O55:H7 (n=7), O76:H51, O84:H2, O86:H40, O89:H2, Ont:H19, Ont:H8, Or:[H9], Or:H33 |

| **STEC** ** (n = 180) |
|---|
| O100:NM (n=2), O101 (n=3), O104:H2 (n=3), **O104:H21 (n=4),** O104:H7 (n=2), O105:H18, O109, O110 (n=2), O110:H28, O111:H10, O112ac:H19, O113 (n=2), **O113:H21 (n=4),** O113:H4 (n=3), O115:H18, O116:H28, O117 (n=2), O117:H7 (n=2), O118:H12 (n=3), O125, O126, O126:H8, O128ab:H2, O130:H11, O136 (n=3), O138, O139, O139:H1, O141:[H4], O141ac, O145, O145:H2, O146:H21, O146:H8, O147, O149:H19, O15:H16, O153:H25, O156:H21, O156:H33, O168:H8, O17/77:H41, O171:H-, O171:H2, O172:H21, O174:H2, O174:H21 (n=11), O174:H8 (n=3), O176:H-, O178:H19, O179:H8, O180:H-, O181:H49, O2:H25, O2:H27, O21:H21 (n=3), O22:H16 (n=2), O22:H8 (n=3), O23:H15, O3 (n=2), O40:H21, O40:H8, O41:H7, O46:H38 (n=2), O48:H21, O5:[H19], O53 (n=2), O6 (n=7), O6:H10 (n=2), O6:H34 (n=2), O68:H12 (n=2), O73:H18, O74:H42, O75:H8, O76, O76:H19 (n=2), O77 (n=2), O79, O79:H48, O8,O104:H10, O8:H10, O8:H19 (n=7), O8:H8, O85:H11 (n=2), O9,O104:H12 (n=2), **O91 (n=5), O91:H10 (n=3),** O91:H14 (n=2), **O91:H21 (n=6),** O92,O107:H-, O92,O107:H48, O96:H19, Ont:H-, Ont:H7, Or:H12, Or:H16, Or:H29, Or:H33, Or:H48, OX178:H19, OX183:H18, OX184:H11, OX185:H28, OX187:Hbev, OX3:H-, OX3:H2, OX3:H21, OX7:H16 |

| **Apathogenic EC** (n = 51) |
|---|
| O103 (n=3), O103:H8, O104:H21, O104:H7, O111, O111:H12, O121:H45, O132:H18, O141:H2, O145:H2, O153:H12, O156:H47, O156:H56, O157 (n=9), O157:H10, O157:H12, O157:H15, O157:H19, O157:H25, O157:H27, O157:H42, O157:H43, O22/083, O26 (n=4), O26:H21/32, O26:H32 (n=6), O4:H5, O45:H7, O6:H4, O62:H30 (n=2), O8,O104:H45, O88 |

| |
|---|
| For each serotype, n=1 unless otherwise stated. * Including EHEC derivatives as described in (Bugarel et al. 2010). EHEC from top 7 noted in bold. ** Including atypical EHEC noted in bold |

High throughput real-time PCR testing was performed as described in Example 1 above.

Primers and probes used for PCR amplifications of the genetic markers *ecs1763, espM1, espM2, espN, espO1-1, espW, espX7, terE and iha* are listed in Table VI. Primers and probes for the detection of *stx1l, stx2, eae,* and *nleB* were described previously (Bugarel et al. 2010). Amplification of the genes *stx1, stx2, eae* and *nleB* were used as internal controls and for group assignment purposes.

**Table VI**

| | Forward primer, reverse primer and probe sequences (5' - 3') | SEQ ID NO: | Location within sequence AE005174 |
|---|---|---|---|
| terE | GCCGTTACCATCTATGATGC | 65 | 1104959 - 1104978 |
| | TGTAAACGCGCATGAAGCTG | 66 | 1105034 -1105015 |
| | [FAM] - AGCGCGTAAACAAAACTTCGGCATGGTG - [BHQ] | 67 | 1104982 - 1105009 |
| iha | AGTGGTACGGGTAAAACCG | 68 | 1108400 - 1108382 |
| | AGTATCAGCGTGTAACTGGC | 69 | 1108330 - 1108349 |
| | [FAM] - TGGAAATCAGCATCCGAGGAATGCC - [BHQ] | 70 | 1108375 - 1108351 |
| espX7 | TTTATCCTGTCACACAGCAGAGTG | 71 | 1664648 - 1664671 |
| | ACAAAACCCAGTTATAAGCCAAATCG | 72 | 1664746 -1664721 |
| | [FAM] - TCATCATCCCAGTCAGGCTTCCCAGAGC - [BHQ] | 73 | 1664717 - 1664690 |
| espN | GACATATTTGTTTATGTCATCAGGAGCGG | 46 | 1666890 - 1666918 |
| | CCTCAGGATATGGATGGCCTACTGGC | 47 | 1667017 - 1666992 |
| | [FAM] - AATGCTCTCGGCAATCGAATCCTTGACTC - [BHQ] | 56 | 1666991 - 1666963 |
| espO1-1 | CATGTTGTTGATGTAAGTATGCAG | 74 | 1671948 - 1671971 |
| | AAGTTCACAAGTACATTACCCGG | 75 | 1672034 -1672012 |
| | [FAM] - ACGCTGGCTTGTCTTTTATGGGACCG - [BHQ] | 76 | 1671979 - 1672004 |
| ecs1763 | GACAGTCTTCGATTGCCTTTGC | 77 | 1854693 -1854672 |
| | GCAGATTTGCATCATTTTCTAAATTCAC | 78 | 1854608 - 1854635 |
| | [FAM] - AGAGCCTTGTTGATGCCCTGTTTCGTTC - [BHQ] | 79 | 1854664 - 1854637 |
| espM1 | GCGCTCTATCCGCTTTAATGTTAAC | 48 | 2275378 - 2275354 |
| | CCATCCATGAATATCTTTAGTACTCTGC | 49 | 2275291 - 2275318 |
| | [FAM] - TGCTTACCGTCTCCAGTATACAGCCGCT - [BHQ] | 57 | 2275320 - 2275347 |
| espM2 | AAGTTCTATTAATCATGTRATAATGGGG | 80 | 3547051 - 3547024 |
| | ACTTGTCYGCAAGCAAGTTTGCTATG | 81 | 3546921 - 3546946 |
| | [FAM] - GCTCTTTGTGATRGTATGAGCCGGGATG - [BHQ] | 82 | 3546996 - 3546969 |
| espW | CTTAGGAGAGCGAGACGTAAGAATG | 83 | 3548909 - 3548933 |
| | TGAATAAAGATACTCACCTAACTCTG | 84 | 3549011 - 3548986 |
| | [FAM] - TTCCTTCCTGTTATCTTATCTGTATCATCAG - [BHQ] | 85 | 3548980 - 3548950 |

### Results

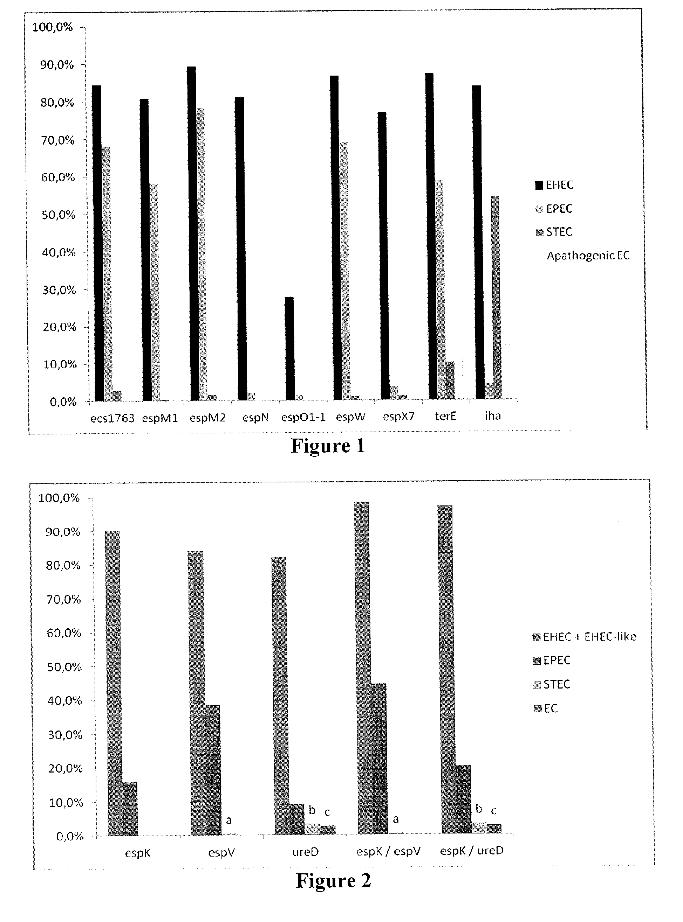

The results are shown in Figure 1 and in Tables VII to X

### Distribution of the genetic markers among various E. coli pathogroups

The distribution of the different genetic markers (*ecs1763, espM1, espM2, espN, espO1-1, espW, espX7, terE* and *iha*) among the different *E. coli* pathogroups is shown in Figure 1. The genetic markers *espN, espX7* and *espO1-1* were detected almost exclusively in EHEC strains with few exceptions in EPEC strains. EPEC strains giving a positive reaction for at least one of these markers are reported in Table IX. The genetic markers *espN, espX7* and *espO1-1* were not present in apathogenic *E. coli* and STEC strains, except for two STEC strains (O113:H4 and O156:H21) which tested positive for *espX7* (Table X).

The genetic markers *ecs1763, espM1 espM2, espW* and *terE* were detected mostly in EHEC and EPEC strains. Except for *terE,* which was found in 10% of STEC strains and 11.8% of apathogenic *E. coli,* these markers were rarely detected in STEC and apathogenic *E. coli.* STEC and apathogenic *E. coli* strains giving a positive reaction for at least one of these genetic markers are reported in Table X. Regarding the prevalence of the gene *iha,* it was detected in a large proportion of EHEC strains (83.8 %), as well as 54.4 % of STEC strains. *Iha* was found less frequently in apathogenic *E. coli* (15.7 %) and EPEC strains (4.3%).

By combining detection of *espN* with *espM1* most of the EHEC strains were tested positive. Thus, 313/327 EHEC (95.7%) and 82/137 EPEC (59.9%) gave a positive result for *espN* and/or *espM1.* These two genetic markers were not detected in STEC and apathogenic *E. coli* strains, except for one *stx-* and *espM1-positive* O156:H21 strain.

### Distribution of the genetic markers in enterohaemorrhagic E. coli

The distribution of each genetic marker *ecs1763, espM1, espM2, espN, espO1-1, espW, espX7, terE* and *iha* was significantly different according to EHEC serotypes (Tables VII and VIII). Interestingly, the genetic markers *ecs1763* and *espM1* have the same distributions. They were both detected in all EHEC O111:[H8], O121:[H19], O145:[H28], in 98.4% of O157:[H7] and in 90.4% of O26:[H11] EHEC strains. These two genes were absent in EHEC O103:[H2]. The genes *espM2* and *espW,* located in close proximity in the *E. coli* genome, were detected with very similar frequencies in EHEC strains. Thus, all EHEC O157:[H7] and O111:[H8] gave a positive result for *espM2* and *espW.* Among EHEC O26:[H11] 98.2% gave a positive result for *espM2* and 97.6% for *espW.* Both genes were detected with the same frequency in O103:[H2] (66.7%) and in O121:[H19] (75%). Both genes were also absent from EHEC O145:[H28], except for one strain which tested positive for *espM2.* Finally, *espM2* and *espW* were detected in 75.7% and 56.8% of the new emerging EHEC respectively. Other genetic markers such as *espN* and *espX7* are also closely related in regard to their distribution in EHEC. The genes *espN* and *espX7* have comparable distributions in EHEC O145:[H28] (100% *espN,* 100% *espX7),* O103:[H2] (100% *espN,* 94.4% *espX7*)*,* O111:[H8] (100% *espN,* 89.5% *espX7*)*,* O157:[H7] (85.5% *espN,* 82.3% *espX7*)*,* O26:[H11] (76.6% *espN,* 72.5% *espX7*) and O121:[H19] (75% *espN,* 75% *espX7*)*.*

At the opposite, the distribution of *terE* and *iha* differ significantly. The gene *terE* was found in all EHEC O121:[H19] and O145:[H28], but was less frequent in EHEC O157:[H7] (93.5%), O111:[H8] (89.5%), O26:[H11] (87.4%), O103:[H2] (50%) and other EHEC (83.8%). The gene *iha* was detected in all O145:[H28] strains, but was detected less frequently in other EHEC serotypes, with frequencies ranging from 25% to 96.8%.

The gene *espO1-1* appeared to be preferentially associated to certain serotypes. Indeed, it was found in 100% of O111:[H8]. It was less frequent in O145:[H28] (93.8%) and O157:[H7] (85.5%) strains. This gene was virtually absent from the other EHEC serotypes, being found in only 0 to 5.4% of other EHEC serotypes.

By combining detection of *espN* with *espM1* most of the EHEC serotypes associated with EHEC infections in humans were tested positive. Thus, all EHEC O157:[H7], O103:[H2], O111:[H8], O121:[H19] and O145:[H28] strains gave a positive result for *espN* and/or *espM1,* while 97% of O26:[H11] were tested positive.

### Distribution of the genetic markers in enteropathogenic E. coli

The distribution in EPEC strains of each genetic marker varied significantly according to EPEC serotypes. The distribution of the genetic markers in EPEC O26:[H11] was significantly different from the distribution of these genetic markers in the other EPEC serotypes (Table IX).

The genes *espN, espX7* and *espO1-1* were overall very rare in EPEC strains (less than 5%) and completely absent from EPEC O26:[H11]. The genetic markers *ecs1763, espM1 espM2, espW* and *terE* were found in most of EPEC strains. However, while they were largely found in EPEC O26:[H11] (between 85.2% and 97.5%), they were much less frequently represented in other EPEC (between 8.8% for *espM1* to 50.9% for *espM2*)*.*

Significant differences in the EPEC strains were observed when combining detection of *espN* and *espM1.* Thus, the genes *espN* and/or *espM1* although frequently found in EPEC O26:[H11] (92.6%) were rarely (14%) detected in other EPEC strains.

**Table VII: Distribution of the different genetic markers in EHEC and EHEC-like strains**

| **Target gene** | **O157:H7 (n = 62)** | **0103:H2 (n=18)** | **026:H11 (n=167)** | **0111:H8 (n = 19)** | **O121:H19 (n=8)** | **0145:H28 (n = 16)** | **other EHEC (n = 37)** |
|---|---|---|---|---|---|---|---|
| e*cs1763* | 98.4% | 0.0% | 90.4% | **100.0%** | **100.0%** | 100.0% | 56.8% |
| *espM1* | 98.4% | 0.0% | 90.4% | **100.0%** | **100.0%** | 100.0% | 24.3% |
| *espM2* | **100.0%** | 66.7% | 98.2% | **100.0%** | 75.0% | 6.3% | 75.7% |
| *espN* | 85.5% | **100.0%** | 76.6% | **100.0%** | 75.0% | **100.0%** | 67.6% |
| *espO1-1* | 85.5% | 0.0% | 1.2% | **100.0%** | 0.0% | 93.8% | 5.4% |
| *espW* | **100.0%** | 66.7% | 97.6% | 1**00.0%** | 75.0% | 0.0% | 56.8% |
| *espX7* | 82.3% | 94.4% | 72.5% | 89.5% | 75.0% | **100.0%** | 62.2% |
| *terE* | 93.5% | 50.0% | 87.4% | 89.5% | **100.0%** | **100.0%** | 83.8% |
| *iha* | 96.8% | 44.4% | 94.0% | 94.7% | 25.0% | **100.0%** | 35.1% |
| *espN*/*espM1** | **100.0%** | **100.0%** | 97.0% | **100.0%** | **100.0%** | **100.0%** | 75.7% |

| | | | | | | | |
|---|---|---|---|---|---|---|---|
| **espN*/*espM1* represent strains that are positive for at least *espN* or *espM1.* | | | | | | | |

**Table VIII: EHEC and EHEC-like strains negative for the different genetic markers**

| **Target gene** | **Gene absent in EHEC and EHEC-like of serotypes:** |
|---|---|
| *ecs1763* | **O103:H2 (n=18),** O15:H2, **O157:H7,** O165:H25, O172:[H25], O177, **O26:H11 (n=16), O45:H2 (n=2),** O49:H16, O5 (n=8), OX186:[H2] |
| *espM1* | **O103:H2 (n=18),** O103:H25 (n=2), O15:H2, O156:H25 (n=10), **O157:H7,** O165:H25, **O26:H11 (n=16), O45:H2 (n=2),** O5 (n=8), O55, O55:H7, Or:H16, OX186:[H2] |
| *espM2* | **O103:H2 (n=6), O121:H19 (n=2), O145:H28 (n=15),** O156:H25 (n=6), O165:H25, O172:[H25], **O26:H11 (n=3),** O49:H16 |
| *espN* | **O121:H19 (n=2),** O123:H11, O15:H2, O156:H25 (n=6), **O157:H7 (n=9),** 0177 (n=2), **O26:H11 (n=39),** Or:H16, OX186:[H2] |
| *espO1-1* | **O103:H2 (n=18),** O103:H25 (n=2), O118:[H16] (n=3), 0118:H+, **O121:H19** (n=8), O123:H11, **O145:H28,** O15:H2, O156:H25 (n=10), O157:H7 (n=9), 0165:H25, O172:[H25], 0177 (n=2), **O26:H11 (n=165), O45:H2 (n=2),** O49:H16, O5 (n=8), Or:H16,OX186:[H2] |
| *espW* | **O103:H2 (n=6), O121:H19 (n=2), O145:H28 (n=16),** O156:H25 (n=10), 0165:H25, O172:[H25], **O26:H11 (n=4),** O49:H16, O55, O55:H7, Or:H16 |
| *espX7* | **O103:H2,** O111:H8 (n=2), **O121:H19 (n=2),** O123:H11, O15:H2, O156:H25 (n=6), **O157:H7 (n=11),** O172:[H25], O177 (n=2), **O26:H11 (n=46),** O5, Or:H16, OX186:[H2] |
| *terE* | **O103:H2 (n=9), 0111:H8 (n=2),** O15:H2, **O157:H7 (n=4),** O165:H25, O172:[H25], **O26:H11 (n=21),** O55, O55:H7, Or:H16 |
| *iha* | **O103:H2 (n=10),** O103:H25, **O111:H8, O121:H19** (n=6), O15:H2, O156:H25 (n=10), **O157:H7 (n=2),** O165:H25, O172:[H25], **O26:H11 (n=10), O45:H2 (n=2),** O5 (n=8) |

| | |
|---|---|
| For each serotype, n=1 unless otherwise stated. EHEC from top 7 serotypes noted in bold. | |

**Table IX: Distribution of the different genetic markers in EPEC strains**

| **Target gene** | **Presence in EPEC O26:H11 (n=81)** | **Presence in EPEC non 026 (n = 57)** | **Gene present in EPEC strains** |
|---|---|---|---|
| *ecs1763* | 92.6% | 33.3% | O111:H11, O111:H47, O127:H6, O128:H2, O128ac:H2, O156, O156:H25 (n=3), O156:H8, **O26:H11 (n=75),** O26:H31, O55:H7 (n=6), O84:H2, O89:H2 |
| *espM1* | 92.6% | 8.8% | O111:H11, O111:H25, O111:H47, O127:H6, O156:H8, **O26**:**H11 (n=75)** |
| *espM2* | 97.5% | 50.9% | O111:H11, O111:H2, O111:H47, O113, O127:H6, 0128:H2 (n=2), O15:H2, O156, O156:H1 (n=2), O156:H25 (n=3), O156:H8, O157, O157:H2, O157:H26, **O26:H11 (n=79),** O26:H31, O26:H34, O55:H7 (n=7), O76:H51, Or:[H9] |
| *espN* | 0.0% | 5.3% | O156:H25, O55:H7, Or:H33 |
| *espO1-1* | 0.0% | 3.5% | O55:H7, Or:H33 |
| *espW* | 97.5% | 28.1% | O111:H11, O111:H2, O113, O127:H6, O128:H2 (n=2), O15:H2, O156, O156:H1, O156:H25, O156:H8, O157:H2, **O26:H11 (n=79),** O26:H31, O26:H34, O76:H51, Or:[H9] |
| *espX7* | 0.0% | 8.8% | O127:H6, O156:H25, O55:H7 (n=2), Or:H33 |
| *terE* | 85.2% | 21.1% | O103:H21, O111:H11, O111:H47, O127:H6, O156:H25 (n=3), O156:H8, O157, **O26:H11 (n=69),** O26:H31, O26:H34, Or:[H9] |
| *iha* | 1.2% | 8.8% | O111:H11, O111:H47, O127:H6, **O26:H11,** O55:H7, Or:[H9] |

| | | | |
|---|---|---|---|
| For each serotype, n=1 unless otherwise stated. EPEC 026 are noted in bold. | | | |

**Table X: Distribution nf the different genetic markers in STEC and non pathogenic E. coli strains**

| **Target gene** | **Gene present in STEC* strains** |
|---|---|
| *ecs1763* | O136, O139:H1, O156:H21, O53, O8:H19 |
| *espM1* | O156:H21 |
| *espM2* | O156:H21, O53, Or:H33 |
| *espN* | - |
| *espO1-1* | - |
| *espW* | O156:H21, Or:H33 |
| *espX7* | O113:H4, O156:H21 |
| *terE* | O100:NM, O111:H10, O113, **O113:H21,** O113:H4, O138, O139, O141ac, O147, O168:H8, O171:H-, O178:H19, O21:H21, O40:H8, O48:H21, Ont:H-, Or:H33, OX178:H19 |
| *iha* | O100:NM, O104:H7, O111:H10, O112ac:H19, O113 (n=2), **O113:H21 (n=2),** O113:H4 (n=3), O117, O117:H7 (n=2), O118:H12 (n=3), O128ab:H2, O138, O139, O146:H21, O147, O153:H25, O156:H21, O156:H33, O168:H8, O171:H-, O171:H2, O172:H21, O174:H21 (n=11), O174:H8 (n=3), O176:H-, O178:H19, O181:H49, O21:H21 (n=2), O22:H16 (n=2), O22:H8 (n=2), O40:H21, O46:H38 (n=2), O48:H21, O5:[H19], 053 (n=2), O6 (n=7), O6:H10 (n=2), O6:H34 (n=2), O74:H42, O75:H8, O85:H11 (n=2), **O91 (n=5), O91:H10 (n=3),** O91:H14 (n=2), **O91:H21 (n=6),** O96:H19, Ont:H-, Ont:H7, Or:H29, OX178:H19, OX3:H-, OX3:H21, OX7:H16 |

| **Target gene** | **Gene present in non pathogenic *E. coli* strains** |
|---|---|
| *ecs1763* | O157 (n=2), O157:H19 |
| *espM1* | - |
| *espM2* | - |
| *espN* | - |
| *espO1-1* | - |
| *espW* | - |
| *espX7* | - |
| *terE* | O111, O157, O157:H19, O26, O62:H30 (n=2) |
| *iha* | O111, O111:H12, O157:H10, O157:H19, O157:H25, O22/083, O62:H30 (n=2) |

| | |
|---|---|
| For each serotype, n=1 unless otherwise stated. * Including atypical EHEC (noted in bold). | |

### 2) Genetic markers espK, espV and UreD

The production of Shiga toxin (Stx) by enterohemorrhagic *E. coli* (EHEC) is the primary virulence trait responsible for Hemorrhagic colitis (HC) and Hemolytic Uremic Syndrome (HUS), but many *E. coli* strains that produce Stx (STEC) do not cause HC and HUS. Besides the ability to produce one or more types of Shiga toxins, STEC strains associated with human infections harbor other factors which might be used to distinguish STEC strains constituting a severe risk for human health from STEC strains that are not associated with severe and epidemic disease. Among these factors, the urease activity *(ureD)* and EspK *(espK),* an effector that has been found to be involved in persistence of EHEC O157:H7 in the intestines of orally inoculated calves (Vlisidou et al. 2006), might be used to identify pathogenic STEC strains. Genome sequencing of few EHEC strains (EHEC O157:H7, O111, O103 and O26) seems to corroborate this hypothesis and pointed out other genetic markers, such as *espV* whose role in disease has not been evaluated. The gene *espV* has been found in the genomes of these EHEC strains but its prevalence in other *E. coli* pathogroups has not been documented yet. The distribution of the genes *ureD, espK* and *espV* in various *E. coli* pathogroups was examined to assess their association with STEC strains with high virulence for humans.

### Materials and methods

*E. coli* strains (n = 987) investigated in this study are reported in Table XI. They were divided into Shiga-toxin producing *E. coli* or STEC (n = 176), enteropathogenic *E. coli* or EPEC (n = 322), enterohaemorrhagic *E. coli* or EHEC (n = 345) and apathogenic *E. coli* (n = 144), using the same criteria as above.

**Table XI**

| **EHEC*** (n = 345) |
|---|
| O103:H2 (n=39), O103:H25 (n=6), O111:H8 (n=49), O118:[H16] (n=4), O118:H+, O119:H25 (n=5), **O121:H19** (n=13), O123:H11, O127:H8s, O145, O145:[H28] (n=29), O156:H21, O156:H25 (n=11), O157:[H7] (n=75), O165:H25 (n=2), O172:H25 (n=2), O172:NM, O177 (n=2), O177:[H25], O182:[H25], **O26:[H11]** (n=77), O3, O45:H2 (n=2), O49:H16, O5 (n=4), O5:H- (n=5), O5:NM (n=2), O55, O55:H7, O76:H51, O84:H2, Ont:[H2], Ont:H25, Or:H16, OX186:[H2] |

| **EPEC** (n = 322) |
|---|
| O100:[H25] (n=2), O102:H19, O103:H21, O103:H8, O108:H9 (n=6), 0109:H25, 0111, 0111:B4, O111:H11, O111:H19 (n=3), O111:H2 (n=13), O111:H25 (n=2), O111:H47, O111:H9 (n=3), O113:H6 (n=2), O114:H2 (n=6), O114:H49 (n=5), O115:H38 (n=3), O117:H25, O117:H40b (n=3), O118:H5, O118:H8a (n=3), O119:[H25], O119:[H52], O119:H2 (n=3), O119:H6 (n=4), O119:H8 (n=2), O119:H9, O119s:H2, O123/04:H45 (n=2), O123:H25, O125:H6, O125ac:H6 (n=3), O126:H27, O126:H6, O127, O127:H19, O127:H40 (n=4), O127:H40b (n=2), O127:H6 (n=2), O128:[H2] (n=12), O128:H8, O128ac:H2, O142:H34, O142:H6 (n=3), O145:H34 (n=5), O15:H11, O15:H2 (n=4), O153:H14, O156, O156:[H8] (n=7), O156:H1 (n=2), O156:H25 (n=3), O157, 0157:[H45] (n=5), O157:H16 (n=5), O157:H2, O157:H26 (n=2), O157:H39, O168:[H33], O177:H26, O186:[H45], O2:[H40] (n=2), O2:H40b, O2:H8, O21:H25, O22:H7, O26:[H11] (n=39), O26:H34, O28:H28 (n=4), O3:H40b, O3:H5, O3:H8a (n=3), O37:H10, O4:H16, O45, O45:H7, O45:H9, O49:[H10], O49:H-, O49:H10, O5:H11, O51:H49 (n=3), O55:[H51], O55:[H7] (n=26), O55:H6 (n=5), O62:H9, O63:H6 (n=2), O65:H25, O66:H8/8a, O69:[H2], O69:H16 (n=2), O70/086:H2, O70:H11 (n=5), O71:H40b, O76:H41, O76:H7 (n=5), O80:[H2] (n=3), O84:[H2], O86:[H34], O86:H11 (n=2), O86:H34 (n=3), O86:H40, 086:H8 (n=4), O86:H8a, O88:H8a,O89:H2, O9:H10, OK8:H10, Ont:[H10], Ont:[H52], Ont:[H6), Ont:H11, Ont:H14, Ont:H2 (n=2), Ont:H21 (n=4), Ont:H24, Ont:H26, Ont:H40b, Ont:H6, Ont:H7, Ont:Hnt, Or:[H9] (n=2), Or:H10, Or:H33, Or:H40b (n=2), Or:H6, Or:H7, Or:H8a, OX177:H11 (n=2), OX177:H6 |

| **STEC** (n = 176)** |
|---|
| O100:NM (n=2), O101:H- (n=3), O104:H2 (n=2), O104:H21 (n=2), O104:H7, O105:H18, 0109:H-, O110, O110:H28, O111, O111:H10, O113 (n=2), O113:H21 (n=4), O113:H4 (n=3), O115:H18 (n=2), O116:H28, O117 (n=2), O117:H7 (n=2), O118:H12 (n=3), O125, O126, 0126:H8, O128ab:H2, O130:H11, O136 (n=3), O138, O139, O139:H1, O141:[H4], O141ac, O146:H21, O146:H28 (n=2), O146:H8, O147, O149:H19, O15:H16, O153:H25 (n=3), O165:H11, O168:H8, O17/77:H41, O171:H-, O171:H2, O172:H21, O174:[H21] (n=8), O174:H2, O174:H21 (n=3), O174:H8 (n=4), 0176:H-, O178:H19, O179:H8, O181:H49, 02:H25, O2:H27, O21:H21 (n=3), O22/083, O22:H16 (n=2), O22:H8 (n=3), O23:H15, O3, O39:H48, O40:H21, O40:H8, O46:H38 (n=2), O48:H21, O5:[H19], O5:H-, O53 (n=2), O6 (n=7), O6:H10 (n=2), O6:H34 (n=2), O68:H12, O73:H18, O74:H42, O75:H8, O76, O76:H19 (n=3), O77 (n=2), O79, O79:H48, O8:H10, O8:H19 (n=6), O8:H8 (n=2), O85:H11, O86, O88:H25, O91 (n=6), O91:[H21] (n=5), O91:H10 (n=3), O91:H14 (n=2), O92,0107:H-, O92,O107:H48, O96:H19, Ont:H-, Ont:H7, Or:H12, Or:H16, Or:H29, Or:H33, Or:H4, Or:H48, OX178:H19, OX185:H28, OX187:Hbev, OX3:H-, OX3:H2, OX3:H21, OX7:H16 |

| **Apathogenic EC (n = 144)** |
|---|
| O103 (n=2), O103:H8, O104:H-, O104:H21 (n=2), O104:H7 (n=2), O110, O111:H12, O111:H21, O121:[H45], O126 (n=33), O126:H11, O126:H27 (n=3), O127 (n=8), O127:H10, O127:H21, O132:H18, O141:H2, O142 (n=12), O145:H2 (n=2), O150:H8, O153:H12, O156:H33, O156:H47, O156:H56, O157 (n=10), O157:H10, O157:H12, O157:H15, O157:H19, O157:H25, O157:H27, O157:H42, O157:H43, O180:H-, O26 (n=5), O26:H21/32, O26:H32 (n=6), O4:H5, O41:H7, 045:H7, O55 (n=9), O55:H19, O55:H21, O6:H4, O62:H30 (n=2), O8,O104:H10, O8:K9:H45, O86 (n=6), O86/O125ac, O86:H2, O86:H27, O88, O9:K9:H12 (n=2), OX183:H18, OX184:H11 |

| |
|---|
| For each serotype, n=1 unless otherwise stated. * Including EHEC-like strains (or EHEC derivatives) as described in (Bugarel et al. 2010), strains from top 7 serotypes are noted in bold ** Including atypical EHEC serotypes noted in bold |

High-throughput real-time PCR amplifications were performed as described above.

Primers and FAM-labeled TaqMan® probes used for PCR amplifications of *stx1, stx2,* and *eae* were previously described (Bugarel al. 2010). Primers and probes used for targeting *ureD, espK* and *espV are* listed in Table XII below.

**Table XII**

| Target gene^{a} | Forward primer, reverse primer and probe sequences (5' - 3') | SEQ ID NO: | Location within sequence AE005174 |
|---|---|---|---|
| espK (Z1829) | GCAGRCATCAAAAGCGAAATCACACC | 44 | 1673422-1673397 |
| | TCGTTTGGTAACTGTGGCAGATACTC | 45 | 1673312 -1673338 |
| | [6FAM]-ATTCAGATAGAAGAAGCGCGGGCCAG-[BHQ] | 55 | 1673395 - 16673370 |
| espV (Z1387) | TCAGGTTCCTCGTCTGATGCCGC | 50 | 1295446 -1295424 |
| | CTGGTTCAGGCCTGGAGCAGTCC | 51 | 1295360 -1295382 |
| | [6FAM]-CTTGCAACACGTTACGCTGCCGAGTATT-[BHQ] | 58 | 1295422 - 1295395 |
| ureD (Z1142) | GCAATAATTGACTCTGATTGCC | 86 | 1078824 -1078845 |
| | GCTGCTGCGGTAAAATTTACT | 87 | 1078892 -1078872 |
| | [6FAM] -TACGCTGATCACCATGCCTGGTGC-[BHQ] | 88 | 1078847 - 1078870 |

| | | | |
|---|---|---|---|
| a) Numbering as in EDL933 | | | |

### Results

### Distribution of the genes espK, espV and ureD among various E. coli pathogroups

The relationship between the genetic markers investigated in this work and the various *E. coli* pathogroups is shown in Figure 2. The genetic markers *espK* and *espV* were detected only in EHEC and EPEC strains. They were not present in apathogenic *E. coli* and STEC strains, with the exception of only one STEC strain (Or:H33) which tested positive for *espV.* The genes *espK* and *esp V* were more frequent in EHEC (90.1 % for *espK* and 84.1 % for *espV*) than in EPEC (15.8 % for *espK* and 38.5 % for *espV*)*.* The gene *ureD* was much more common in EHEC (82.0 %) than in EPEC (9.3 %), STEC (3.4 %) and avirulent *E. coli* (2.8 %).

Among the non-EHEC strains, EPEC represent most of the strains that tested positive for at least one of the genetic markers *espK, espV* and *ureD.* EPEC strains giving a positive reaction for at least one of these genetic markers are reported in Table XIII. Among the 322 EPEC strains tested in this study, 15.8 % were positive for *espK,* 38.5% were positive for *esp V* and 9.3% tested positive for *ureD.*

By combining detection of *espK* with either *esp V* or *ureD* most of the EHEC strains were tested positive (Figure 2). 339/345 EHEC (98.3 %) and 143/322 EPEC (44.4 %) gave a positive results for *espK* and/or *espV.* No strain belonging to the other pathogroups was tested positive for any of these two genetic markers. The *stx-* and *espV*-positive Or:H33 strain was the unique exception. 335/345 EHEC (97.10 %), 65/322 EPEC (20.2 %), 6/176 (3.4 %) STEC and 4/144 (2.8 %) avirulent *E. coli* gave a positive results for *espK* and/or *ureD.*

**Table XIII: EPEC strains positive for esipK, espV and ureD**

| **EPEC strains positive for *espK*** (n = 51) |
|---|
| O100:[H25], O111, O111:H11, O111:H19 (n=3), O111:H47, O111:H9 (n=3), O114:H2 (n=6), O115:H38, O117:H25, O119:[H25], O119:H2, O119:H8 (n=2), O119:H9, O125ac:H6, O128:[H2], O156:H25, O157, O157:H2, O157:H26 (n=2), O22:H7, O28:H28 (n=3), O37:H10, 049:[H10], O55:[H7] (n=2), O76:H41, 076:H7 (n=4), O80:[H2] (n=3), O84:[H2], Ont:[H10], Ont:H26, Or:[H9], Or:H33 |

| **EPEC strains positive for *espV*** (n = 124) |
|---|
| O100:[H25] (n=2), O102:H19, O103:H8, O108:H9 (n=6), O109:H25, O111:H19 (n=3), O111:H2 (n=13), O111:H9 (n=3), O114:H2 (n=6), O115:H38 (n=3), O117:H25, O118:H8a, O119:H8, O119:H9, O123/O4:H45 (n=2), O125ac:H6, O126:H27, O128:[H2] (n=3), O128ac:H2, O15:H11, O156:[H8] (n=2), O156:H25 (n=3), O157, O157:[H45] (n=5), O157:H16 (n=5), O157:H26 (n=2), O177:H26, O186:[H45], O2:[H40] (n=2), O2:H40b, O26:[H11] (n=11), O28:H28 (n=4), O3:H8a, O37:H10, O4:H16, O49:H-, O5:H11, O62:H9, O70:H11 (n=5), O76:H7, O84:[H2], O86:H11 (n=2), O86:H8 (n=2), O86:H8a, O88:H8a, O89:H2, OK8:H10, Ont:[H10], Ont:[H52], Ont:H2, Ont:H26, Ont:H7, Ont:Hnt, Or:[H9], Or:H10, Or:H33, Or:H6, Or:H7, OX177:H11 (n=2) |

| **EPEC strains positive for *ureD*** (n = 30) |
|---|
| O100:[H25] (n=2), O109:H25, O111, O111:B4, O111:H11, O111:H19, O111:H47, O111:H9 (n=3), O117:H25, O119:[H25], O119:H9, O156:H25 (n=3), O37:H10, O49:H-, O49:H10, O70:H11 (n=5), O84:[H2], Ont:[H10] Ont:Hnt, Or:[H9], Or:H7 |

| **EPEC strains positive for *espK* and/or *espV*** (n = 143) |
|---|
| O100:[H25] (n=2), O102:H19, O103:H8, O108:H9 (n=6), O109:H25, O111, O111:H11, O111:H19 (n=3), O111:H2 (n=13), O111:H47, O111:H9 (n=3), O114:H2 (n=6), O115:H38 (n=3), O117:H25, O118:H8a, O119:[H25], O119:H2, O119:H8 (n=2), O119:H9, O123/O4:H45 (n=2), O125ac:H6, O126:H27, O128:[H2] (n=3), O128ac:H2, O15:H11, O156:[H8] (n=2), O156:H25 (n=3), O157, O157:[H45] (n=5), O157:H16 (n=5), O157:H2, O157:H26 (n=2), O177:H26, O186:[H45], O2:[H40] (n=2), O2:H40b, O22:H7, O26:[H11] (n=11), O28:H28 (n=4), O3:H8a, O37:H10, O4:H16, O49:[H10], O49:H-, O5:H11, O55:[H7] (n=2), O62:H9, O70:H11 (n=5), O76:H41, O76:H7 (n=5), O80:[H2] (n=3), O84:[H2], O86:H11 (n=2), O86:H8 (n=2), O86:H8a, O88:H8a, O89:H2, OK8:H10, Ont:[H10], Ont:[H52], Ont:H2, Ont:H26, Ont:H7, Ont:Hnt, Or:[H9], Or:H10, Or:H33, Or:H6, Or:H7, OX177:H11 (n=2) |

| **EPEC strains positive for espK and/or ureD (n = 65)** |
|---|
| O100:[H25] (n=2), O109:H25, O111, O111:B4, O111:H11, O111:H19 (n=3), O111:H47, O111:H9 (n=3), O114:H2 (n=6), O115:H38, 0117:H25, O119:[H25], O119:H2, O119:H8 (n=2), O119:H9, O125ac:H6, O128:[H2], O156:H25 (n=3), O157, O157:H2, O157:H26 (n=2), O22:H7, O28:H28 (n=3), O37:H10, O49:[H10], O49:H-, O49:H10, O55:[H7] (n=2), O70:H11 (n=5), O76:H41, O76:H7 (n=4), O80:[H2] (n=3), O84:[H2], Ont:[H10], Ont:H26, Ont:Hnt, Or:[H9], Or:H33, Or:H7 |

### Distribution of the genes espK, espV and ureD in enterohaemorrhagic E. coli

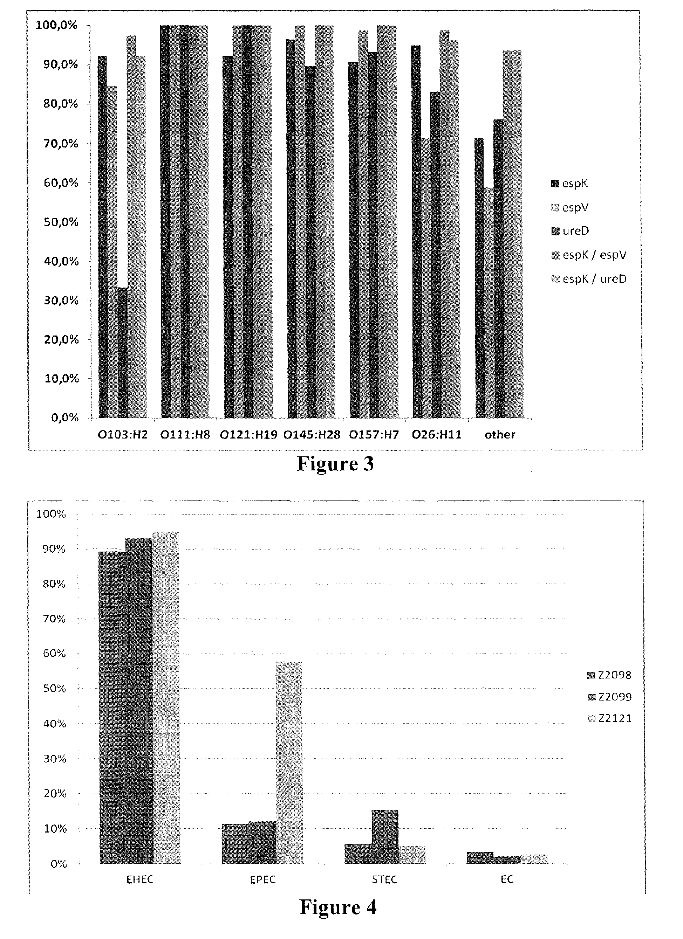

The distribution of the genes *espK, espV* and *ureD* in EHEC is reported in Figures 2 and 3. Among the 345 EHEC strains tested in this study, 90.1 % were positive for *espK,* 84.1% were positive for *esp V* and 82.0% tested positive for *ureD.* EHEC strains giving a negative reaction for at least one of these genetic markers are reported in Table XIV. The distribution of *espK, esp V* and *ureD* was significantly different according to EHEC serotypes (Figure 3).

The gene *espK* was detected in all EHEC O111:[H8] strains, but was less frequent in O145:[H28] (96.6 %), O26:[H11] (94.8 %), O121:[H19] (92.3 %), O103:[H2] (92.3 %), 0157:[H7] (90.7 %), and other EHEC (71.4 %).

The gene *espV* was detected in all EHEC O111:[H8], O121:[H19] and O145:[H28] strains, but was less frequent in O157:[H7] (98.7 %), O103:[H2] (84.6 %), O26:[H11] (71.4 %), and other EHEC (58.7 %).

The gene *ureD* was detected in all EHEC O111:[H8] and O121:[H19] strains, but was less frequent in O157:[H7] (93.3 %), 0145:[H28] (89.7 %), O26:[H11] (83.1 %), O103:[H2] (33.3 %) and other EHEC (76.2 %).

By combining detection of *espK* with either *espV* or *ureD* most of the EHEC serotypes associated with EHEC infections in humans were tested positive (Figure 3). Thus, all EHEC 0157:[H7], O145:[H28], O111:[H8] and O121:[H19] strains gave a positive results for *espK* and/or *espV* 98.7 % of O26:[H11] and 97.5 % of O103:[H2] gave a positive result for *espK* and/or *espV.* Data were very similar when testing *espK* with *ureD.* Hence, all EHEC O157:[H7], O145:[H28], O111:[H8] and O121:[H19] strains gave a positive results for *espK* and/or *ureD.* 96.1 % of O26:[H11] and 92.3 % of O103:[H2] gave a positive result for *espK* and/or *ureD.*

**Table XIV: EHEC and EHEC-like strains negative for espK, espV and ureD**

| **Target gene** | **Gene absent in EHEC and EHEC-like strains** |
|---|---|
| *espK* | **0103:H2 (n=3),** 0119:H25 (n=4), **0121:H19,** 0127:H8s, **0145:[H28],** 0156:H21, 0156:H25 (n=6), **0157:[H7] (n=7),** 0177:H-, 0177:NM, O182:[H25], **O26:[H11] (n=4),** 03, 076:H51, Ont:H25 |
| *espV* | **0103:H2 (n=6),** O118:[H16] (n=4), 0118:H+, O123:H11, 0127:H8s, 0156:H21, 0156:H25, **0157:[H7], O26:[H11] (n=22),** 03, **045:H2 (n=2),** 05 (n=4), 05:H- (n=5), 05:NM (n=2), 076:H51, Ont:[H2], OX186:[H2] |
| *ureD* | **0103:H2 (n=26),** 0127:H8s, **0145:[H28] (n=3),** 0156:H21, **0157:[H7] (n=5),** 0165:H25 (n=2), 0172:H25 (n=2), O172:NM, **O26:[H11] (n=13),** 03, 049:H16, 055, 055:H7, 076:H51, Ont:[H2], Or:H16, OX186:[H2] |
| *espK*//*espV* | **0103:H2,** 0127:H8s, 0156:H21, **026:[H11],** 03, 076:H51 |
| *espK*//*ureD* | **0103:H2 (n=3),** 0127:H8s, 0156:H21, **O26:[H11] (n=3),** 03, 076:H51 |

| | |
|---|---|
| For each serotype, n=1 unless otherwise stated. EHEC of top 7 are noted in bold. espK//espV represents strains giving a negative result for *espK* and *espV.* espK//ureD represents strains giving a negative result for *espK* and *ureD.* | |

### 3) Genetic markers Z2098, Z2099, and Z2121

Previous studies (Coombes et al., 2008; Imamovic et al, 2010; Bugarel et al., 2011) suggested that type III effectors present in the O-island 57 (OI-57) may be associated with increased virulence and/or pathogenicity of STEC strains. In this study we have selected 3 ORFs located in the OI-57 (Z2098, Z2099, Z2121) and evaluated their distribution of in various *E*. *coli* pathogroups to assess their association with STEC strains with high virulence for humans.

### Materials and methods

*E. coli* strains investigated in this study were those listed in Table XI above High-throughput real-time PCR was performed as described above

Primers and probes for the detection of Z2098, Z2099 and Z2121 were designed for this work and are listed in Table XV below.

**Table XV**

| Target gene^{a} | Forward primer, reverse primer and probe sequences (5' - 3') | SEQ ID NO: | Location within sequence AE005174 |
|---|---|---|---|
| Z2098 | CTGAAAAGAGCCAGAACGTGC | 42 | 1888173-1888193 |
| | TGCCTAAGATCATTACCCGGAC | 43 | 1888308-1888287 |
| | [HEX]TAACTGCTATACCTCCGCGCCG[BHQ] | 54 | 1888286-1888265 |
| Z2099 | TAGCGGGACAATTGTCACGG | 89 | 1889124-1889143 |
| | GTCTTTCGGAGAAACATTCTGCC | 90 | 1889190-1889168 |
| | [HEX]ATATTGATGACAGCGTATGGGCCG[BHQ] | 91 | 1889144-1889167 |
| Z2121 | GATGGCAGATAATAACGAAGCAAC | 92 | 1904798-1904821 |
| | CAGCCGTTGAAGCATCAGCG | 93 | 1904915-1904896 |
| | [HEX]AGCAGCATTCTTGCAGACCCTTACGG[BHQ] | 94 | 1904823-1904848 |

| | | | |
|---|---|---|---|
| a) Numbering as in EDL933 | | | |

### Results

### Association of OI-57 encoded genes with EHEC strains

We analyzed 987 *E*. *coli* strains including EHEC and EHEC-like strains (n=345), EPEC strains (n=322), STEC strains (including atypical EHEC) (n=176) as well as non pathogenic *E*. *coli* (n=144) for the presence of three putative genes (Z2098, Z2099, and Z2121) encoded on the OI-57 genomic island.

The distribution of the three genetic markers in the different *E*. *coli* pathogroups is shown in Figure 4. The two genetic markers Z2098 and Z2099 were frequently detected in EHEC strains (89% for Z2098 and 93% for Z2099). In contrast, they were largely absent from EPEC strains (11% for Z2098 and 12% for Z2099), STEC strains (6% and 15% respectively) and apathogenic *E*. *coli* strains (3% and 2% respectively). Z2121 was significantly associated to both EHEC and EPEC strains (p< 0.001), as it was found in 95% of EHEC strains and 58% of EPEC strains. It was detected in only 5% of STEC strains and 3% of apathogenic *E*. *coli* strains.

The distribution of Z2098 and Z2099 among EHEC serotypes is not uniform (Table XVI). Z2098 was detected in most EHEC of serotypes 0103:[H2], 0145:[H28], O111:[H8], O26:[H11], O121:[H19] and 045:[H2] (EHEC top 6) (94.3%), but was less frequent in O157:[H7] (82.7%) and new emerging EHEC (80.3%). Z2099 was detected in most EHEC top 6 (95.2%) and new emerging EHEC (93.4%). It was detected at different frequencies according to the serotype. Hence, Z2099 was less frequent in O121:[H19] (76.9 %) and 0157:[H7] (86.7%) than in 0103:[H2] (92.3 %), 0111:[H8] (95.9 %), O26:[H11] (97.4 %) and 0145:[H28] (100 %).

EHEC strains giving a negative reaction for Z2098 or Z2099 are reported in Table XVI. The twenty four strains that were Z2099 negative comprised sixteen EHEC strains which belonged to serotypes 0157:[H7] (n=10), 0111:H8 (n=2), 0127:H8s, 055, 055:H7 and 076:H51, as well as eight EHEC-like strains belonging to serotypes 0103:H2 (n=3), O121:H19 (n=3) and O26:H11 (n=2). These twenty four Z2099-negative strains were also negative for Z2098, along with one EHEC-like strain which belonged to serotype O121:H19 and twelve EHEC strains of serotype 0111:H8, O123:H11, 0156:H21, 0157:[H7] (n=3), 0172:[H25] (n=2), 0172:NM, Ont:[H2], Or:H16 and Ox186:[H2].

It is noteworthy that the EHEC and EHEC-like O26:H11 strains, H 19 and CB6706 respectively, which were previously found negative for *espK* (Bugarel et al. 2011) tested positive for Z2098 and Z2099. Concerning the EHEC-like 026:H11 strains, we observed a good correlation between Z2099 or Z2098 and *espK* (data not shown). Among the 33 EHEC-like 026:H11 strains, 31 of them were also positive for Z2099 or Z2098. The two strains that were negative for Z2099 and Z2098 were C810-09 and C314-09. Both strains had the *arcA* allele type 2 but were negative for *nleB* (data not shown).

**Table XVI: Distribution of Z2098 and Z2099 in EHEC and EHEC-like strains**

| Target gene | Presence in EHEC O157:[H7] (n=75) [95% Cl^{‡}] | Presence in EHEC top 6* (n=209) [95% CI^{‡}] | Presence in new emerging EHEC** (n=61) [95% CI^{‡}] | Gene absent in EHEC and EHEC-like strains |
|---|---|---|---|---|
| Z2098 | 82.67% [75.48 - 89.86] | 94.26% [91.61 - 96.91] | 80.33% [71.96 - 88.70] | **0103:H2 (n=3)^{b}, O111:H8 (n=3)^{a}, O121:H19 (n=4)^{b},** O123:H11^{a}, O127:H8s^{a}, O156:H21^{a}, **0157:H7 (n=13)^{a},** 0172:[H25] (n=2)^{a}, O172:NM^{a}, **O26:H11 (n=2)^{b},** O55^{a}, O55:H7^{a}, O76:H51^{a}, Ont:[H2]^{a}, Or:H16^{a}, OX186:[H2]^{a} |
| Z2099 | 86.67% [80.21 - 93.12] | 95.22% [92.79 - 97.64] | 93.44% [88.23 - 98.66] | **0103:H2 (n=3)^{b}, 0111:H8 (n=2)^{a}, O121:H19 (n=3)^{b},** O127:H8s^{a}, **O26:H11 (n=2)^{b},** O55^{a}, O55:H7^{a}, O76:H51^{a} |

| | | | | |
|---|---|---|---|---|
| a) EHEC; b) EHEC-like * EHEC top 6 includes EHEC belonging to serotypes 0103:[H2], 0145:[H28], 0111:[H8], O26:[H11], O121:[H19] and 045:[H2]. ** new emerging EHEC includes all EHEC serotypes different from 0157:[H7] and EHEC top 6. ^{‡} Cl, confidence interval. For each serotype, n=1 unless otherwise stated. Strains from top 7 EHEC serotypes are noted in bold. | | | | |

### Presence of Z2098 and Z2099 in EPEC, STEC and pathogenic E. coli strains

EPEC, STEC and apathogenic *E*. *coli* strains giving a positive reaction with Z2098 and/or Z2098 are reported in Table XVII Among the 176 STEC strains tested, 10 of them (6%) were found positive for Z2098, including several atypical EHEC strains (0104:H21, O113:H21, 091 and 091:H10), and few STEC strains (O110, 0113:H4, 0117, 0136, 0146:H8, and 079). Out of the 176 STEC strains tested, 27 (15%) were found positive for Z2099. These STEC included atypical EHEC strains 0104:H21, 0113:H21, 091 and O91:H10 and STEC strains O105:H18, 0110, O113:H4, O117, O146:H21, 0174:H8, O21:H11, 022:H8, O5:[H19], 05:H-, 06, 074:H42, 075:H8, 076, 076:H19, O85:H11, and Or:H33.

Thirty seven EPEC strains out of 322 (12%) belonging to 22 serotypes were found positive for Z2098. Similarly, 39 EPEC strains (12%) belonging to 23 serotypes were found positive for Z2099. The genetic markers Z2098 and Z2099 were also present in 3% each of the non pathogenic *E*. *coli* tested, including 0104:H7, O110, O132:H18, 0141:H2, and 0157.

**Table XVII: Detection of Z2098 and Z2099 in EPEC, STEC and apathoaenic E. coli strains**

| **EPEC strains positive for Z2098 (n=37)** | **EPEC strains positive for Z2099 (n=39)** | **EPEC strains positive for Z2098 and Z2099 (n=32)** |
|---|---|---|
| 0100:H25, 0108:H9 (n=6), 0109:H25, 0111:H2, 0114:H49, 0115:H38, 0117:H25, 0119:H8, O128:H2, O15:H11, 0156:H25 (n=3), 0156:H8, 0157:H2, 028:H28 (n=4), 045:H9, 05:H11, 062:H9, O70:H11 (n=5), Ont:[H52], Ont:H7, Ont:Hnt, OX177:H11 (n=2) | O100:H25, 0108:H9 (n=6), 0109:H25, 0111, O111:H11, O111:H47, 0117:H25, 0128:H2, O15:H11, 0156:H25 (n=3), 0156:H8, 0157:H2, 028:H28 (n=4), 045:H9, O5:H11, 055:H7 (n=2), 062:H9, O70:H11 (n=5),O84:[H2], Ont:H7, Ont:Hnt, Or:H40b, OX177:H11 (n=2) | O100:H25, 0108:H9 (n=6), O109:H25, 0117:H25, 0128:H2, O15:H11, O156:H25 (n=3), 0156:H8, 0157:H2, 028:H28 (n=4), 045:H9, O5:H11, 062:H9, O70:H11 (n=5), Ont:H7, Ont:Hnt, OX177:H11 (n=2) |

| **STEC strains* positive for Z2098 (n=10)** | **STEC strains* positive for Z2099: (n=27)** | **STEC strains* positive for Z2098 and Z2099 (n=5)** |
|---|---|---|
| **0104:H21,** 0110, **0113:H21,** 0113:H4, 0117, 0136, 0146:H8, 079, **091, 091:H10** | **0104:H21,** 0105:H18, 0110, **0113:H21,** 0113:H4 (n=2), O117, O117, O146:H21, 0174:H8 (n=3), 021:H21, 022:H8, O5:[H19], 05:H-, 06, 074:H42, 075:H8, 076, 076:H19 (n=2), O85:H11, **091 (n=3), 091:H10,** Or:H33 | **0104:H21,** 0110, 0113:H4, O117, **O91:H10** |

| **Apathogenic *E*. *coli* strains positive for Z2098 (n=5)** | **Apathogenic *E*. *coli* strains positive for Z2099 (n=3)** | **Apathogenic *E*. *coli* strains positive for Z2098 and Z2099 (n=3)** |
|---|---|---|
| 0104:H7, 0110, O132:H18, O141:H2, O157 | 0104:H7, 0110, O132:H18 | 0104:H7, 0110, O132:H18 |

| | | |
|---|---|---|
| For each serotype, n=1 unless otherwise stated. * Including atypical EHEC (noted in bold) | | |

### Diagnostic application of Z2098 and Z2099 in the detection of typical EHEC strains

For the collection of 987 strains of multiple origins (humans, animals and food) used in this study, the use of Z2098 to detect typical EHEC and EHEC-like strains has a sensitivity of 89.3%, a specificity of 91.9%, a positive predictive value of 85.6% and a negative predictive value of 94.1%. The use of Z2099 to detect typical EHEC and EHEC-like strains has a sensitivity of 93.0%, a specificity of 89.3%, a positive predictive value of 82.3% and a negative predictive value of 96.0%.

### 4) Summary:

The above studies allowed to select five genetic markers (*espM1, espN, espV, espK* and *Z2098*) useful for detecting EHEC strains belonging to the six major serotypes of EHEC reported worldwide in human infections. The distribution of these different genetic markers has been investigated among the different *E*. *coli* pathogroups, allowing to design optimal sub-combinations of these markers. The results of these studies are summarized below.
- By combining detection of *espN* with *espM1* most of the EHEC strains were tested positive. Thus, 257/281 EHEC (91.5%) and 84/300 EPEC (28%) gave a positive result for *espN* and/or *espM1.* These two genetic markers were not detected in STEC and apathogenic *E*. *coli* strains.
- By combining detection of *espK* with *Z2098* most of the EHEC strains were tested positive. Thus, 276/281 EHEC (98.2%) and 74/300 EPEC (24.7%) gave a positive result for *espK* and/or *Z2098.* These two genetic markers were less frequently detected in STEC (6/155, 3.9%) and apathogenic *E*. *coli* (3/119, 2.5%) strains.
- By combining detection of *espK* with *espV* most of the EHEC strains were tested positive. 339/345 EHEC (98.3 %) and 143/322 EPEC (44.4 %) gave a positive results for *espK* and/or *espV.* No strain belonging to the other pathogroups was tested positive for any of these two genetic markers. The *stx*- and *espV-*positive Or:H33 strain was the unique exception.
- By combining detection of *espN* with *Z2098* most of the EHEC strains were tested positive. Thus, 267/281 EHEC (95%) and 46/300 EPEC (15.3%) gave a positive result for *espN* and/or *Z2098*. These two genetic markers were less frequently detected in STEC (6/155, 3.9%) and apathogenic *E*. *coli* (3/119, 2.5%) strains.

The distribution of these genetic markers in enterohaemorrhagic *E*. *coli* is summarized in Table XVIII

**Table XVIII. Distribution of the genetic markers in enterohaemorrhagic E. coli**

| Gene association | Top7 EHEC serotypes | O103:H2 | O111:H8 | O121:H19 | O103:H28 | O157:H7 | O26:H11 | O45:H2 | Other EHEC (new emerging EHEC) | Total EHEC |
|---|---|---|---|---|---|---|---|---|---|---|
| *espN*/*espM1* | 226/231 (97.8%) | 27/30 (90%) | 17/17 (100%) | 8/8 (100%) | 24/24 (100%) | 75/75 (100%) | 74/76 (97.4%) | 1/1 | 31/50 (62%) | 257/281 (91.5%) |
| *Z2098*/*espK* | 229/231 (99.1%) | 29/30 (96.7%) | 17/17 (100%) | 8/8 (100%) | 24/24 (100%) | 74/75 (98.7%) | 76/76 (100%) | 1/1 | 47/50 (94%) | 276/281 (98.2%) |
| *espV*/*espK* | 281/283 (99.3%) | 38/39 (97.5%) | 49/49 (100%) | 13/13 (100%) | 28/28 (100%) | 75/75 (100%) | 76/77 (98.7%) | 2/2 | 58/62 (93.5%) | 339/345 (98.3%) |
| *espN*/*Z2098* | 224/231 (97%) | 29/30 (96.7%) | 17/17 (100%) | 5/8* (62.5%) | 24/24 (100%) | 74/75 (98.7%) | 74/76 (97.4%) | 1/1 | 43/50 (86%) | 267/281 (95%) |

| | | | | | | | | | | |
|---|---|---|---|---|---|---|---|---|---|---|
| * The 0121:[H19] strains that tested negative for *espN* and *22098* were also *stx* negative. These strains may be EHEC derivatives having lost their *stx* genes or may belong to EPEC pathogroup. | | | | | | | | | | |

The gene *espN* was detected in all EHEC O111:[H8] and O145:[H28] strains, but was less frequent in O103:[H2] (90 %), O157:[H7] (86.7 %), 026:[H11] (68.4 %), O121:[H19] (62.5 %), and other EHEC (52 %).

The gene *espM1* was detected in all EHEC 0111:[H8], O121:[H19] and 0145:[H28] strains, but was less frequent in 0157:[H7] (97.3 %), O26:[H11] (90.8 %), O103:[H2] (3.3 %), and other EHEC (24%).

The gene *espK* was detected in all EHEC O111:[H8] strains, but was less frequent in O145:[H28] (96.6 %), O26:[H11] (94.8 %), O121:[H19] (92.3 %), O103:[H2] (92.3 %), 0157:[H7] (90.7 %), and other EHEC (71.4 %).

The gene *espV* was detected in all EHEC O111:[H8], O121:[H19] and O145:[H28] strains, but was less frequent in O157:[H7] (98.7 %), O103:[H2] (84.6 %), O26:[H11] (71.4 %), and other EHEC (58.7 %).

Z2098 was detected in most EHEC of serotypes O103:[H2], O145:[H28], 0111:[H8], O26:[H11], O121:[H19] and 045:[H2] (EHEC top 6) (94.3%), but was less frequent in 0157:[H7] (82.7%) and new emerging EHEC (80.3%).
- By combining detection of *espN* with *espM1* most of the EHEC serotypes associated with EHEC infections in humans were tested positive. Thus, all EHEC 0157:[H7], O111:[H8], O121:[H19] and O145:[H28] strains gave a positive result for *espN* and/or *espM1,* while 97.4% of O26:[H11] and only 90% of O103:[H2] were tested positive.
- By combining detection of *espK* with *Z2098* most of the EHEC serotypes associated with EHEC infections in humans were tested positive. Thus, all EHEC O111:[H8], O121:[H19], O26:[H11] and O145:[H28] strains gave a positive result for *espK* and/or *Z2098,* while 98.7% of O157:[H7], and 96.7% of O103:[H2] were tested positive.
- By combining detection of *espK* with *espV* most of the EHEC serotypes associated with EHEC infections in humans were tested positive. Thus, all EHEC O157:[H7], 0145:[H28], O111:[H8] and O121:[H19] strains gave a positive results for *espK* and/or *espV.* 98.7 % of O26:[H11] and 97.5 % of O103:[H2] gave a positive result for *espK* and/or *espV.*
- By combining detection of *espN* with *Z2098* most of the EHEC serotypes associated with EHEC infections in humans were tested positive. Thus, all EHEC O145:[H28] and O111:[H8] strains gave a positive results for *espN* and/or *Z2098.* 98.7 % of O157:[H7], 97.4 % of O26:[H11] and 96.7 of O103:[H2] gave a positive result for *espN* and/or *Z2098.* Only 5/8 (62.5%) O121:[H19] were tested positive for *espN* and/or *Z2098.* It is noteworthy that the O121:[H19] strains that tested negative for *espN* and *Z2098* were also *stx* negative. These strains may be EHEC derivatives having lost their *stx* genes or may belong to EPEC pathogroup. There is no possibility to definitely define the pathogroup of these strains in absence of specific discriminative genetic markers.

The distribution of these genetic markers in enteropathogenic *E*. *coli* is summarized in Table XIX.

**Table XIX. Distribution of the genetic markers among various E. coli pathogroups**

| Gene association | Top7 EHEC serotypes | EPEC | EPEC 026:[H11] | EPEC non O26:[H11] | STEC | Avirulent *E*. *coli* |
|---|---|---|---|---|---|---|
| *espN*/*espM1* | 226/231 (97.8%) | 84/300 (28%) | 34/38 (89.5%) | 50/262 (19%) | 0/155 (0%) | 1/119 (0.8%) |
| *Z2098*/*espK* | 229/231 (99.1%) | 74/300 (24.7%) | 0/38 (0%) | 74/262 (28.2%) | 6/155 (3.9%) | 3/119 (2.5%) |
| *espV*/*espK* | 281/283 (99.3%) | 143/322 (44.4%) | 11/39 (28.2%) | 132/283 (46.6%) | 1/176 (0.5%) | 0/144 (0%) |
| *Z2098*/*espN* | 224/231 (97%) | 46/300 (15.3%) | 0/38 (0%) | 46/262 (17.6%) | 6/155 (3.9%) | 3/119 (2.5%) |

The distribution in EPEC strains of each genetic marker varied significantly according to EPEC serotypes. The distribution of the genetic markers in EPEC O26:[H11] was significantly different from the distribution of these genetic markers in the other EPEC serotypes.

The gene *espN,* was very rare in EPEC strains (5.3%) and completely absent from EPEC O26:[H11]. The genetic marker *espM1* was largely found in EPEC O26:[H11] (34/38, 89.5%), and was much less frequently represented in other EPEC (45/262, 17.2%). In final the association of the genes *espN* and/or *espM1* although frequently found in EPEC O26:[H11] (89.5%) was rarely detected in other EPEC (19%).

Among the EPEC strains tested in this study, 15.8 % were positive for *espK* and 38.5% were positive for *espV.* In final the association of the genes *espK* and/or *espV* was less frequently found in EPEC O26:[H11] (28.2%) than in other EPEC (46.6%).

Among the EPEC strains tested in this study, 15.8 % were positive for *espK* and 12 % were positive for *Z2098.* In final the association of the genes *espK* and/or *Z2098* although found in EPEC non-O26:[H11] (28.2%) was never detected in EPEC O26:[H11] (0%).

The genes *espN,* was very rare in EPEC strains (5.3%) and completely absent from EPEC 026:[H11]. The genetic marker *Z2098* was found in only 12 % of EPEC. In final the association of the genes *espN* and/or *Z2098* although found in EPEC non-O26:[H11] (17.6%) was never detected in EPEC O26:[H11] (0%).

### Conclusion

We used a high throughput PCR approach to explore the virulome of different *E*. *coli* pathogroups in an attempt to identify genetic traits that would characterize pathogenic STEC strains. The distribution of five genetic markers (*espM1, espN, espV, espK* and *Z2098*) was investigated in a large panel of *E*. *coli* comprising EHEC, EPEC, STEC and apathogenic *E*. *coli* strains. The distribution of these genetic markers varied between the *E*. *coli* pathogroups and according to the serotypes.

Overall, four associations of genes (*espN*/*espM1,* Z2098/*espK, espV*/*espK* and *Z2098*/*espN*) were shown the best combinations for detecting EHEC strains belonging to the six major serotypes of EHEC reported worldwide in human infections. These findings showed that using this relevant combinations of genes most of the EHEC strains were tested positive while only a subset of the EPEC strains were cross reacting. Also, only very minor STEC and avirulent *E*. *coli* strains cross-reacted when using such an approach.

### REFERENCES

Beutin, L., S. Jahn, and P. Fach. 2009. Evaluation of the 'GeneDisc' real-time PCR system for detection of enterohaemorrhagic Escherichia coli (EHEC) 026, 0103, 0111, 0145 and 0157 strains according to their virulence markers and their 0- and H-antigen-associated genes. J. Appl. Microbiol. 106(4): 1122-1132.
Bugarel M, Martin A, Fach P, Beutin L. 2011. Virulence gene profiling of enterohemorrhagic (EHEC) and enteropathogenic (EPEC) Escherichia coli strains: a basis for molecular risk assessment of typical and atypical EPEC strains. BMC Microbiol. Jun 21;11:142.
Bugarel M, Beutin L, Scheutz F, Loukiadis E, Fach P. 2011. Identification of genetic markers for differentiation of Shiga toxin-producing, enteropathogenic, and avirulent strains of Escherichia coli 026. Appl Environ Microbiol. Apr;77(7):2275-81.
Bugarel M, Beutin L, Martin A, Gill A, Fach P. 2010. Micro-array for the identification of Shiga toxin-producing Escherichia coli (STEC) seropathotypes associated with Hemorrhagic Colitis and Hemolytic Uremic Syndrome in humans. Int J Food Microbiol. Sep 1;142(3):318-29.
Bugarel M, Beutin L, Fach P. 2010. Low-density macroarray targeting non-locus of enterocyte effacement effectors (nle genes) and major virulence factors of Shiga toxin-producing Escherichia coli (STEC): a new approach for molecular risk assessment of STEC isolates. Appl Environ Microbiol. Jan;76(1):203-11.
Cergole-Novella MC, Nishimura LS, Dos Santos LF, Irino K, Vaz TM, Bergamini AM, Guth BE. 2007. Distribution of virulence profiles related to new toxins and putative adhesins in Shiga toxin-producing Escherichia coli isolated from diverse sources in Brazil. FEMS Microbiol Lett. Sep;274(2):329-34. Epub 2007 Jul 25.
Chang, C. 1991 "Branched DNA Amplification Multimers for the Sensitive, Direct Detection of Human Hepatitis Viruses," Nucleic Acids Symposium Series, no. 24: 197-200.
Compton, J. 1991 "Nucleic Acid Sequence-Based Amplification," Nature 350, no. 6313: 91-92.
Coombes BK, Wickham ME, Mascarenhas M, Gruenheid S, Finlay BB, Karmali MA. 2008. Molecular analysis as an aid to assess the public health risk of non-O157 Shiga toxin-producing Escherichia coli strains. Appl Environ Microbiol. Apr;74(7):2153-60.
Creuzburg K, Middendorf B, Mellmann A, Martaler T, Holz C, Fruth A, Karch H, Schmidt H. 2011. Evolutionary analysis and distribution of type III effector genes in pathogenic Escherichia coli from human, animal and food sources. Environ Microbiol. Feb;13(2):439-52.
Frank C, Werber D, Cramer JP, Askar M, Faber M, an der Heiden M, Bernard H, Fruth A, Prager R, Spode A, Wadl M, Zoufaly A, Jordan S, Kemper MJ, Follin P, Muller L, King LA, Rosner B, Buchholz U, Stark K, Krause G; HUS Investigation Team. Epidemic profile of Shiga-toxin-producing Escherichia coli 0104:H4 outbreak in Germany. N Engl J Med. 2011 Nov 10;365(19):1771-80
Gault G, Weill FX, Mariani-Kurkdjian P, Jourdan-da Silva N, King L, Aldabe B, Charron M, Ong N, Castor C, Mace M, Bingen E, Noel H, Vaillant V, Bone A, Vendrely B, Delmas Y, Combe C, Bercion R, d'Andigne E, Desjardin M, de Valk H, Rolland P. Outbreak of haemolytic uraemic syndrome and bloody diarrhoea due to Escherichia coli O104:H4, southwest France, June 2011. Euro Surveill. 2011 Jun 30;16(26).
Imamovic L, Tozzoli R, Michelacci V, Minelli F, Marziano ML, Caprioli A, Morabito S. 2010. OI-57, a genomic island of Escherichia coli O157, is present in other seropathotypes of Shiga toxin-producing E. coli associated with severe human disease. Infect Immun. Nov;78(11):4697-704.
Karmali, M. A., M. Mascarenhas, S. Shen, K. Ziebell, S. Johnson, R. Reid-Smith, J. Isaac-Renton, C. Clark, K. Rahn, and J. B. Kaper. 2003. Association of genomic 0 island 122 of Escherichia coli EDL 933 with verocytotoxin-producing Escherichia coli seropathotypes that are linked to epidemic and/or serious disease. J. Clin. Microbiol. 41: 4930-4940.
Levine, M. M. 1987. Escherichia coli That Cause Diarrhea - Enterotoxigenic, Enteropathogenic, Enteroinvasive, Enterohemorrhagic, and Enteroadherent. J. Infect. Dis. 155: 377-389.
Mackay, I. 2007. Real-time PCR in Microbiology, from diagnosis to characterization. Caister Academic Press, Norfolk, UK.
Nataro, J. P. and J. B. Kaper. 1998. Diarrheagenic Escherichia coli. Clinical Microbiol. Rev. 11: 142-201.
Notomi, T., Okayama, H., Masubuchi, H., Yonekawa, T., Watanabe, K., Amino, N., and Hase, T. 2000 Loop-Mediated Isothermal Amplification of DNA. Nucleic Acids Research 28, no. 12: E63.
Scheutz F, Nielsen EM, Frimodt-Moller J, Boisen N, Morabito S, Tozzoli R, Nataro JP, Caprioli A. Characteristics of the enteroaggregative Shiga toxin/verotoxin-producing Escherichia coli 0104:H4 strain causing the outbreak of haemolytic uraemic syndrome in Germany, May to June 2011. Euro Surveill. 2011 Jun 16;16(24).
Spears KJ, Roe AJ, Gally DL. 2006. A comparison of enteropathogenic and enterohaemorrhagic Escherichia coli pathogenesis. FEMS Microbiol Lett. Feb;255(2):187-202.
Struelens MJ, Palm D, Takkinen J. Enteroaggregative, Shiga toxin-producing Escherichia coli 0104:H4 outbreak: new microbiological findings boost coordinated investigations by European public health laboratories. Euro Surveill. 2011 Jun 16;16(24).
Tobe T, Beatson SA, Taniguchi H, Abe H, Bailey CM, Fivian A, Younis R, Matthews S, Marches O, Frankel G, Hayashi T, Pallen MJ. 2006. An extensive repertoire of type III secretion effectors in Escherichia coli 0157 and the role of lambdoid phages in their dissemination. Proc Natl Acad Sci USA. Oct 3;103(40):14941-6.
Vlisidou I, Marchés 0, Dziva F, Mundy R, Frankel G, Stevens MP. 2006. Identification and characterization of EspK, a type III secreted effector protein of enterohaemorrhagic Escherichia coli O157:H7. FEMS Microbiol Lett. Oct;263(1):32-40.
Walker, G., Fraiser, M., Schram, J., Little, M., Nadeau, J., and Douglas P. Malinowski, D. 1992 Strand Displacement Amplification—An Isothermal, In Vitro DNA Amplification Technique, Nucleic Acids Research 20, no. 7: 1691-1696.

## Claims

1. A method for identifying the serotype(s) of enterohemorrhagic *Escherichia coli* (EHEC) suspected to be present in a sample, wherein said method comprises detecting the presence or the absence, in said sample or DNA isolated therefrom, of the following *E. coli* CRISPR sequences:
a) CRISPR sequences for identifying EHEC 0157:[H7] wherein said CRISPR sequences are selected among
- the CRISPR sequences SEQ ID NO: 1, SEQ ID NO: 2, and SEQ ID NO: 3, wherein the presence of one or more of said CRISPR SEQ ID NO: 1-3 is indicative of the presence of EHEC O157:[H7]; and/or
- the CRISPR sequence SEQ ID NO: 4, wherein the presence of said CRISPR sequence is indicative of the presence of EHEC 0157:[H7];
b) a CRISPR sequence for identifying EHEC O145:[H28], wherein said CRISPR sequence is the sequence SEQ ID NO: 5, and wherein the presence of said CRISPR sequence is indicative of the presence of EHEC O145:[H28]; and
c) a CRISPR sequence for identifying EHEC 0111:[H8], wherein said CRISPR sequence is the sequence SEQ ID NO: 6, and wherein the presence of said CRISPR sequence is indicative of the presence of EHEC O111:[H8]; and
d) a CRISPR sequence for identifying EHEC O121:[H19], wherein said CRISPR sequence is the sequence SEQ ID NO: 7, and wherein the presence of said CRISPR sequence is indicative of the presence of EHEC O121:[H19]; and
e) a CRISPR sequence for identifying EHEC O103:[H2] and/or EHEC 045:[H2], wherein said CRISPR sequence is the sequence SEQ ID NO: 8, and wherein the presence of said CRISPR sequence is indicative of the presence of EHEC O103:[H2] and/or of EHEC 045:[H2]; and
f) a CRISPR sequence for identifying EHEC O104:[H4], wherein said CRISPR sequence is the sequence SEQ ID NO: 9, and wherein the presence of said CRISPR sequence is indicative of the presence of EHEC 0104:[H4]; and
g) a CRISPR sequence for identifying EHEC O26:[H11], wherein said CRISPR sequence is the sequence SEQ ID NO: 10, and wherein the presence of said CRISPR sequence is indicative of the presence of EHEC O26:[H11].

2. A method of claim 1, wherein said method comprises performing a PCR assay on said sample or DNA isolated therefrom with a combination of primers targeting said CRISPR sequences.

3. A method of claim 2, wherein said combination of primers comprises:
a) primers for detecting EHEC 0157:[H7], wherein said primers consist of:
- a set of primers targeting both the CRISPR sequences SEQ ID NO: 1 and SEQ ID NO: 2, wherein said primers are defined by the following sequences:
GGGAACACAAACCGAAACACA (SEQ ID NO: 11)
CTTAGTGTGTTCCCCGCGC (SEQ ID NO: 12) and
- a set of primers targeting the CRISPR sequence SEQ ID NO: 3 wherein said primers are defined by the following sequences:
GAACACTTTGGTGACAGTTTTTGT (SEQ ID NO: 13);
CTTAGTGTGTTCCCCGCGC (SEQ ID NO: 14),
wherein the presence of an amplification product for at least one of said sets of primers is indicative of the presence of EHEC O157:[H7]; and/or:
- a set of primers targeting the CRISPR sequence SEQ ID NO: 4, wherein said primers are defined by the following sequences:
GAACACAAACCGAAACACACG (SEQ ID NO: 15)
ATAAACCGTCACCAAAACAGTG (SEQ ID NO: 16),
wherein the presence of an amplification product for said set of primers is indicative of the presence of EHEC O157:[H7]; and
b) primers for detecting EHEC O145:[H28], wherein said primers consist of:
- a set of primers targeting the CRISPR sequence SEQ ID NO: 5, wherein said primers are defined by the following sequences:
GAACTTGAGCCCTGCCAGAA (SEQ ID NO: 17)
ACCGCGATCTTTTCCTACCTG (SEQ ID NO: 18),
wherein the presence of an amplification product for said set of primers is indicative of the presence of EHEC 0145:[H28]; and
c) primers for detecting EHEC O111:[H8], wherein said primers consist of:
- a set of primers targeting the CRISPR sequence SEQ ID NO: 6, wherein said primers are defined by the following sequences:
GTGACCGCCTGTACACGC (SEQ ID NO: 19)
CGGATATTTGGGCGTAATACC (SEQ ID NO: 20)
CTGCCGCGAGTGGTTTCAC (SEQ ID NO: 21),
wherein the presence of an amplification product for at least one of primers pairs SEQ ID NO: 19 and SEQ ID NO: 20 or SEQ ID NO: 19 and SEQ ID NO: 21 is indicative of the presence of EHEC O111:[H8]; and
d) primers for detecting EHEC O121:[H19], wherein said primers consist of:
- a set of primers targeting the CRISPR sequence SEQ ID NO: 7, wherein said primers are defined by the following sequences:
CGGGGAACACTACAGGAAAGAA (SEQ ID NO: 22)
GGCGGAATACAGGACGGGTGG (SEQ ID NO: 23),
wherein the presence of of an amplification product for said set of primers is indicative of the presence of EHEC O121:[H19]; and
e) primers for detecting EHEC O103:[H2] and/or EHEC 045:[H2], wherein said primers consist of:
- a set of primers targeting the CRISPR sequence SEQ ID NO: 8, wherein said primers are defined by the following sequences:
GAGTCTATCAGCGACACTACC (SEQ ID NO: 24)
AACCGCAGCTCGCAGCGC (SEQ ID NO: 25),
wherein the presence of an amplification product for said set of primers is indicative of the presence of EHEC 0103:[H2] and/or of EHEC 045:[H2]; and
f) primers for detecting EHEC O104:[H4], wherein said primers consist of:
- a set of primers targeting the CRISPR sequence SEQ ID NO: 9, wherein said primers are defined by the following sequences:
GGAACTCACCGAGCGCCG (SEQ ID NO: 26);
GCCTTTGCAGCGTCTTTCCGATC (SEQ ID NO: 27);
wherein the presence of an amplification product for said set of primers is indicative of the presence of EHEC 0104:[H4]; and
g) primers for detecting EHEC O26:[H11], wherein said primers consist of:
- two sets of primers targeting the CRISPR sequence SEQ ID NO: 10, wherein the first primers set is defined by the following sequences:
ACAATCGTGTGTAAATTCGCGG (SEQ ID NO: 28)
GATAAACCGTGGTACGGAACA (SEQ ID NO: 29) and the second said primers set is defined by the following sequences:
TGAAACCACTCGCGGCAGAT (SEQ ID NO: 30);
ATAAACCGATCTCCTCATCCTC (SEQ ID NO: 31);
wherein the presence of an amplification product for at least one of the said sets of primers is indicative of the presence of EHEC O26:[H11].

4. A method for predicting whether a sample contains EHEC of at least one of EHEC O157:[H7], O145:[H28], O103:[H2], O111:[H8], O121:[H19], O26:[H11] and 045:[H2] serotypes, wherein said method comprises the detection of at least one of the following combinations of target genes:
- *Z2098* and at least one of *espK* and *espN;*
- *espN* and *espM1;*
- *espK* and *espV.*

5. A method of claim 4, wherein said method comprises performing a PCR assay on said sample or DNA isolated therefrom with a combination of primers comprising a set of primers derived from *Z2098* and a set of primers derived from at least one of *espK* and *espN* and detecting the presence or the absence of an amplification product for each set of primers of said combination.

6. A method of claim 4, wherein said method comprises performing a PCR assay on said sample or DNA isolated therefrom with a combination of primers comprising a set of primers derived from *espN* and a set of primers derived from *espM1* and detecting the presence or the absence of an amplification product for each set of primers of said combination.

7. A method of claim 4, wherein said method comprises performing a PCR assay on said sample or DNA isolated therefrom with a combination of primers comprising a set of primers derived from *espK* and a set of primers derived from *espV* and detecting the presence or the absence of an amplification product for each set of primers of said combination.

8. A method of any of claims 4 to 7, which further comprises performing a PCR assay on said sample or DNA isolated therefrom with a combination of primers comprising a set of primers derived from *stx1* and a set of primers derived from *stx2* and detecting the presence or the absence of an amplification product for each set of primers of said combination.

9. A method of any of claims 1 to 3, which comprises a previous step for predicting whether said sample contains enterohemorrhagic *Escherichia coli* (EHEC) of at least one of EHEC 0157:H7, 0145:H28, 0103:H2, 0111:H8, O121:H19, O26:H11 and 045 :H2 serotypes, wherein said previous step is carried out by a process according to any of claims 2 to 6.

10. A kit for the identication of the serotype(s) of enterohemorrhagic *Escherichia coli* (EHEC), comprising the sets of primers defined in claim 3, and optionally the probes for detecting the amplification products for each of said set of primers.

11. A kit of claim 10, further comprising at least one combination of set of primers selected among:
- a set of primers targeting *Z2098,* and a set of primers targeting *espK* or a set of primers targeting *espN,* and optionally the probes for detecting the amplification products for each of said set of primers;
- a set of primers derived from *espN* and a set of primers targeting *espM1* and optionally probes for detecting the amplification products for each of said set of primers;
- a set of primers derived from *espK* and a set of primers targeting *espV* and optionally probes for detecting the amplification products for each of said set of primers.

12. A kit of claim 11, further comprising a set of primers targeting *stx1* and *stx2,* and optionally a probe for detecting the amplification product from *stx1* and a probe for detecting the amplification product from *stx2.*
